# EUROPEAN PATENT SPECIFICATION

(11) **EP 4 678 733 B1**
(45) Date of publication and mention of the grant of the patent: **29.07.2026**
(21) Application number: 24821464.5
(22) Date of filing: 18.10.2024
(51) Int. Cl.: C12N 1/20, C12N 1/205, C12P 3/00, A01N 63/20, A01N 59/02, C05D 9/02, C05F 11/08, A01P 21/00

(54) **BACTERIAL STRAIN CAPABLE OF OXIDIZING ELEMENTAL SULPHUR AND USE THEREOF FOR STIMULATING PLANT GROWTH**
BAKTERIENSTAMM ZUR OXIDATION VON ELEMENTAREM SCHWEFEL UND VERWENDUNG DAVON ZUR STIMULIERUNG DES PFLANZENWACHSTUMS
SOUCHE DE BACTÉRIE CAPABLE D'OXYDER LE SOUFRE ÉLÉMENTAIRE ET SON UTILISATION POUR FAVORISER LA CROISSANCE DE PLANTES

(30) Priority: 20.10.2023 ES 202330869
(43) Date of publication of application: 14.01.2026
(73) Proprietor: Azufrera y Fertilizantes Pallares S.A.U., 43120 Tarragona Constanti (ES)
(72) Inventor: GARCIA SECO DE HERRERA, Daniel, 43120 Tarragona Constantí (ES); COMAS MICOLAU, Cristina, 43120 Tarragona Constantí (ES); MONTAÑO SALMERON, Manel, 43120 Tarragona Constantí (ES); AROLA-ARNAL, Anna, 43120 Tarragona Constantí (ES); PEIX GELDART, Alvaro, 43120 Tarragona Constantí (ES); VAZQUEZ FERNANDEZ, Guillermo, 43120 Tarragona Constantí (ES)
(74) Representative: Isern Patentes y Marcas S.L.
(86) International application number: PCT/ES2024/070637
(87) International publication number: WO 2025/083314

(56) References cited:
- WO-A1-2020/170244
- US-B2- 7 329 532
- DATABASE EM_STD [online] 2 July 2010 (2010-07-02), UEDA H. ET AL: "Variovorax sp. DC2a-35 gene for 16S rRNA, partial sequence", XP093249046, Database accession no. AB552859
- DATABASE GenBank [online] 2 May 2023 (2023-05-02), AFORDOANYI D.M. ET AL: "DNA-directed RNA polymerase subunit beta [Variovorax paradoxus]", XP093249052, retrieved from https://www.ncbi.nlm.nih.gov/protein/2499395674 Database accession no. WGT62599
- DUTTA S. ET AL: "Complete Genome Sequence of Variovorax paradoxus JBCE486, Which Alleviates the Salt Stress of the Endemic Plant Ulleung-Sanmaneul", MICROBIOLOGY RESOURCE ANNOUNCEMENTS, vol. 12, no. 1, 1 December 2022 (2022-12-01), pages 1 - 2, XP093248589, ISSN: 2576-098X, Retrieved from the Internet <URL:https://journals.asm.org/doi/pdf/10.1128/mra.01027-22> DOI: 10.1128/mra.01027-22
- ZHAO C. ET AL: "Abundance and diversity of sulphur-oxidising bacteria and their role in oxidising elemental sulphur in cropping soils", BIOLOGY AND FERTILITY OF SOILS, vol. 53, no. 2, 1 December 2016 (2016-12-01), pages 159 - 169, XP036135005, ISSN: 0178-2762, [retrieved on 20161201], DOI: 10.1007/S00374-016-1162-0
- CHAUDHARY S. ET AL: "Microbes-mediated sulphur cycling in soil: Impact on soil fertility, crop production and environmental sustainability", MICROBIOLOGICAL RESEARCH, vol. 271, 127340, 24 February 2023 (2023-02-24), pages 1 - 23, XP093249021, ISSN: 0944-5013, DOI: 10.1016/j.micres.2023.127340
- KUMAR S. ET AL: "Biofertilizers: An ecofriendly technology for nutrient recycling and environmental sustainability", CURRENT RESEARCH IN MICROBIAL SCIENCES, vol. 3, 100094, 20 December 2021 (2021-12-20), pages 1 - 26, XP093110445, ISSN: 2666-5174, Retrieved from the Internet <URL:https://www.ncbi.nlm.nih.gov/pmc/articles/PMC8724949/pdf/main.pdf> DOI: 10.1016/j.crmicr.2021.100094
- SCHMALENBERGER A. ET AL: "The role of Variovorax and other Comamonadaceae in sulfur transformations by microbial wheat rhizosphere communities exposed to different sulfur fertilization regimes", ENVIRONMENTAL MICROBIOLOGY, vol. 10, no. 6, 17 February 2008 (2008-02-17), pages 1486 - 1500, XP072196713, ISSN: 1462-2912, DOI: 10.1111/J.1462-2920.2007.01564.X
- SATOLA B. ET AL: "Metabolic characteristics of the species", APPLIED MICROBIOLOGY AND BIOTECHNOLOGY, SPRINGER, BERLIN, DE, vol. 97, no. 2, 29 November 2012 (2012-11-29), pages 541 - 560, XP035165041, ISSN: 1432-0614, DOI: 10.1007/S00253-012-4585-Z
- JAFFER Y. D. ET AL: "A combined approach of 16S rRNA and a functional marker gene, soxB to reveal the diversity of sulphur-oxidising bacteria in thermal springs", ARCHIVES OF MICROBIOLOGY, SPRINGER BERLIN HEIDELBERG, BERLIN/HEIDELBERG, vol. 201, no. 7, 25 April 2019 (2019-04-25), pages 951 - 967, XP036855900, ISSN: 0302-8933, [retrieved on 20190425], DOI: 10.1007/S00203-019-01666-4
- WÜBBELER J. H. ET AL: "The genome of Variovorax paradoxus strain TBEA6 provides new understandings for the catabolism of 3,3'-thiodipropionic acid and hence the production of polythioesters", JOURNAL OF BIOTECHNOLOGY, vol. 209, 11 June 2015 (2015-06-11), pages 85 - 95, XP029250759, ISSN: 0168-1656, DOI: 10.1016/J.JBIOTEC.2015.06.390

## Description

### Technical Field

The present invention relates to the field of microbiology, particularly bacteriology and applications thereof, especially in agriculture and soil restoration. More specifically, the invention relates to the new strain of the genus *Variovorax* capable of oxidising elemental sulphur and to its use for promoting plant growth.

### Background of the Invention

Sulphur is an important nutrient for higher plants. Higher plants absorb sulphur from the soil mainly in the form of sulphate, from where it is transported to the leaves and reduced to sulphite, and then transformed into sulphide by chloroplasts, to be further metabolised to the amino acid cysteine, from which another important amino acid, methionine, is formed. These two amino acids form a structural and functional part of proteins and enzymes, are of vital importance for living organisms and together provide 11-31% of the total organic sulphur in soil.

Therefore, sulphur plays an essential role in the development and growth of plants, and even in the development and growth of the animals that eat them, because, in addition to being involved in the construction of proteins, it is essential for the formation of chlorophyll (as the main constituent of one of the enzymes necessary for its formation), it is essential in the synthesis of oils in plants (especially in crops intended for oil production), and is active in nitrogen metabolism.

Sulphur is a ubiquitous and abundant element in the soil, but its availability is not sufficient to satisfy plant growth. The main sources of terrestrial sulphur are volcanoes and some rocks; marine reservoirs are ocean sediments and seawater. The proportion of sulphur used in agriculture is very small in relation to the amount existing in natural sources and that generated by human activity (fossil fuels and industrial gases).

Like nitrogen or phosphorus, sulphur is an element that is subjected to various processes and transformations in nature, which make up the sulphur cycle.

A few years ago, an important contribution of sulphur to arable soils was the deposition of this element through acid rains, which were caused by the accumulation of sulphur gases in the atmosphere, associated with the excessive use of petroleum products in industrial production, global population explosion, and the intensity of agronomic practices, both in plant cultivation and in livestock breeding. All these factors had in the past a negative impact on the chemical composition and quality of the global atmosphere, leading to the greenhouse effect, which has been a determining factor in global climate change. In response to the adverse effects that this change has caused on ecosystems, legislations that regulate up until now gas emissions into the atmosphere have been created. One of the chemical elements that was considered to be highly polluting was sulphur (S) to which a strict regulation for use has been applied, leading to a significant reduction in sulphur depositions from the atmosphere over the last 20 years.

Reduction in sulphur gas emissions has reduced acid rainfall, creating an imbalance in the sulphur cycle and soil uptake of the element. This deficit, in addition to the continued practice of intensive agriculture, in which applications of organic matter are limited, inevitably leads to sulphur deficiency in agricultural soils. In addition to the above, sulphur-free fertilisers began to be used, a situation which, over the years and in combination with the circumstances described above, has led to a deficiency of this nutrient in arable soils.

Therefore, in recent years, several countries have reported sulphur deficiency in agricultural soils, associating it with the low production of different crops. In China in 1997, it was quantified that 30% of the agricultural soil in that country (around 40 million hectares) was sulphur-deficient, with the deficit being calculated at 1.2 million tonnes per year, and it was projected that the requirement would rise to 2.4 million tonnes per year by 2013.

The lack of sulphur in plants causes an imbalance at the physiological level, which is reflected at the agronomic level, although symptoms may vary between plant species. Generally, deficient plants are smaller and their growth is slow; since sulphur cannot be readily translocated from more mature leaves to young leaves, sulphur deficiency usually shows up first in young leaves. For example, in corn crop, sulphur deficiency appears as interveinal chlorosis in young leaves; in wheat, the whole plant turns pale while the younger leaves are more chlorotic. Furthermore, some plants have more pronounced sulphur needs, as is the case, for example, with plants from the *Brassicaceae* family, such as those of the genus *Brassica* in particular (collard greens, cabbage, broccoli, cauliflower, etc.), which are plants whose crop yields and the quality of the harvested products decrease when they experienced sulphur deficiency during growth.

Sulphur deficiency in arable soils generated the need to incorporate practices of adding sulphur as a fertiliser in soils where intensive agriculture is practised, this need being second only to those practices of adding nitrogen (N), phosphorus (P), and potassium (K).

Some of the agronomic aspects that are improved by using sulphur as a fertiliser are, for example, an increase in the concentration of protein, amino acids, and vitamins in forages, which has a positive impact on the feeding of animals for human consumption. In a study conducted in Iran with wheat of three flour varieties, it was found that sulphur fertilisation increased grain production, protein concentration, and baking quality. In oilseeds, sulphur fertilisation stimulates growth and improves productivity. Evaluations in vegetables have shown that achieving a sulphur-nitrogen balance results in higher quality and uniformity of vegetables, increased cold resistance, increased drought and salt tolerance, improved soil pathogen control, increased decomposition rate of plant waste and green manure.

In that sense, the sufficiency of sulphur in the plant maintains plant productivity by contributing to the efficient use of other essential nutrients such as N, P, and K; it also improves defence against pathogen attack and is even important for maintaining crop quality.

Therefore, the proper use of sulphur leads to a better quality and quantity of food, which would help to counteract the food insecurity that is expected in future years derived from food losses due to climate change, overpopulation, and water scarcity.

Based on the foregoing, it is of vital importance to control the sulphur content of arable soils and, in the event of a deficiency of this element, establish the necessary sulphur addition strategies in order to move towards sufficient food production. Furthermore, the bioavailability of sulphur to plants must be taken into account when designing these strategies appropriately.

The bioavailability of sulphur in soil will depend on multiple factors (including chemical, physical, and biological processes, as well as very important factors such as pH and soil type), which determine the transformations of sulphur forms that cannot be assimilated by plants into sulphate ion, which is the main form in which sulphur can be absorbed by plants.

There are basically two forms of this element in the soil:
- organic form, which represents more than 90% of the total existing reserve, but is not available as such for use by plants, which need the intervention of soil bacteria and fungi for the mobilisation of inorganic sulphate from said organic compounds, but the rate of transformation of organic forms into usable forms does not exceed 2% a year; and
- inorganic form, such as sulphate ions (SO₄²⁻), sulphides (S²⁻), elemental sulphur (S⁰), sulphite (SO²⁻), thiosulphate (S₂O₃²⁻). and tetrathionate (S₄O₆²⁻), that can be found in soils in combination with calcium, magnesium, potassium, sodium, and ammonium.

Of all these forms, as mentioned above, the main form of sulphur in soil that is available for absorption by plants is in the form of sulphate ions. The current availability of free sulphate is not sufficient to satisfy plant growth, especially in terms of availability in agricultural soils, in which the decrease in acid rainfall, the continued practice of intensive agriculture, and the use of sulphur-free fertilisers has led to a deficiency of this nutrient in arable soils.

Due to this need, techniques have been gradually developed to increase the levels of sulphur forms in the soil available for absorption by plants, which can be used by the plants to meet their metabolic needs.

Traditionally, the practices of adding sulphur to soils in agriculture have been carried out using compounds derived mainly from the generation of sulphate ions (the form of sulphur that can be absorbed by roots) and vary from very slightly soluble (agricultural gypsum) to highly soluble (ammonium sulphate, magnesium sulphate, etc.).

Sulphate ion, which is water-soluble, is the form of sulphur that can be assimilated most easily by plant roots although, when found in the soil solution, it is also susceptible to lixiviation to deeper horizons where roots are no longer present, or even being completely removed from the soil profile by deep drainage. Therefore, one of the limitations of the type of sulphate ion precursor-based fertilisers or additives mentioned above is the natural lixiviation that takes place in soils: the movement of nutrients by water that seeps and percolates through the soil, which results in the soil losing its depth. Lixiviation, therefore, causes the movement of soluble or dispersible substances (clay, salts, iron, humus, sulphate ions, etc.) and occurs to a greater extent in humid climates. It causes the upper horizons of the soil to lose their nutritive compounds, washed away by water, and even become more acidic, as insoluble compounds such as aluminium are left behind, and can sometimes even cause toxicity. Large amounts of fertilisers are also lost. Excessive irrigation and the use of fertilisers with high acidity contribute to this phenomenon which, in the case of the addition of sulphur in the form of sulphate ion, results in the loss of said nutrient. Another way of adding sulphur is the addition of elemental sulphur which is usually in the form of insoluble and hydrophobic particles that are difficult to absorb by the roots, and for absorption it has to be transformed naturally and progressively in the soil into sulphate ion. This process of oxidising elemental sulphur, as well as sulphides, sulphites, thiosulphates, or tetrathionates, to give rise to sulphates, can be caused by the action of different microorganisms present in the soil, mainly of the genera *Thiobacillus, Acidithiobacillus, Pseudomonas, Arthrobacter, Bacillus,* and some fungi. Thiobacteria, for example, (such as, for example, *Acidithiobacillus thiooxidans*) present in soil catalyse a natural biochemical reaction in which, formally, sulphuric acid (which can condition alkaline soils) and also energy used in the metabolic process of the thiobacteria themselves are generated:

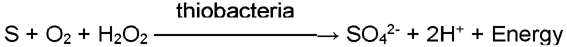

Unlike the use of techniques of adding sulphur in the form of sulphates, the use of elemental sulphur in fertilisation has the added value of progressive transformation into the form. i.e., sulphate, that can be assimilated by plants, with lixiviation losses due to elemental sulphur not being water-soluble being prevented and with sulphate being supplied during practically the entire vegetative cycle, by means of controlled release, determined by the availability of sulphate in the soil. Furthermore, it gives rise to more porous and oxygenated soils, improves the efficiency of use of essential plant nutrients such as nitrogen and phosphorus, contributing to the solubilisation of insoluble phosphorus salts present in soil due to the action of the sulphuric acid formed, also providing an indirect nutritional value as a conditioner for soil (especially alkaline, limestone, and saline soils) and favouring the assimilation of microelements that are absorbed in the form of sulphates (iron, copper, zinc, etc.).

Thus, for instance, Zhao *et al.* (Zhao C. et al., "Abundance and diversity of sulphur-oxidising bacteria and their role in oxidising elemental sulphur in cropping soils", Biology and Fertility of Soils, vol. 53, no. 2, 1 December 2016, pages 159-169), Chaudhary et al. (Chaudhary S. et al., "Microbes-mediated sulphur cycling soil: Impact on soil fertility, crop production and environmental sustainability", Microbiological Research, vol. 271, art. Z no. 127340, 24 February 2023, pages 1-23) and Kumar *et al.* (Kumar S. et al., "Biofertilizers: An ecofriendly technology for nutrient recycling and environmental sustainability", Current Research in Microbial Sciences, vol. 3, art. no. 100094, 20 December 2021, pages 1-26) describe sulphur-oxidising bacteria for use in plant growth stimulation, addressing oxidising elemental sulphur to sulphate. Chaudhary *et al.* mention as important microorganisms involved in sulphur oxidation a group of bacteria belonging to the genus *Acidithiobacillus, Thiobacillus* and heterotrophic bacteria including *Cytobacillus firmus, Enterobacter cloacae, Enterobacter ludwigii, Klebsiella oxytoca, Phytobacter diazotrophicus* and *Pseudomonas stutzeri,* also mentioning greenhouse and field studies were the inoculation of sulphur oxidising bacteria increases crop yield which is higher after the combined inoculation with nitrogen-fixing bacteria. Zhao *et al.* discussed the effects of the presence of sulphur oxidising bacteria in cropping soils, mentioning *Alphaproteobacteria, Betaproteobacteria* and *Deltaproteobacteria* and *Actinobacteria* as genera linked to that activity.

Jaffer *et al.* (Jaffer Y. D. et al:, "A combined approach of 16S rRNA and a functional marker gene, soxB to reveal the diversity of sulphur-oxidising bacteria in thermal springs", Archives of Microbiology, vol. 201, no. 7, 25 April 2019, pages 951-967) discloses studies about sulphur oxidising bacteria present in thermal springs, focusing on soxB, which encodes a sulphate thiohydrolase (sulphate thiol esterase) as a potential marker for their identification. Firmicutes, α-, β-, γ-Proteobacteria and Actinobacteria were dominant in the isolates, although the soxB gene clone library sequences had shown affiliation with the β-, γ- and α-Proteobacteria, *Variovorax* being mentioned among the genera showing homology with the sequence of soxB gene.

The other way of transforming sulphur-containing compounds into soluble forms that can be used by plants, in which microorganisms are involved, is the so-called mineralisation. Mineralisation is the conversion of organic sulphur from residual organic matter from crops and livestock breeding by the action of microbiota present in soil, giving rise to sulphate ion that is available to plants. In contrast, immobilisation is the opposite process in which microbiota transform available sulphur into unavailable organic sulphur. The immobilisation process can also occur when the soil is acidic, which promotes sulphate immobilisation by iron and aluminium oxides, as well as binding to positively charged sites such as clays.

Mineralisation is an important plant process as organic sulphur compounds, and in particular sulphonates and sulphate esters, can constitute more than 90% of the total sulphur present in a soil, especially if the soil has been used for agriculture or livestock breeding. Since the sulphur that is part of said compounds is not available to plants, plants are entirely dependent on soil microorganisms (mainly bacteria and fungi) for their mineralisation.

Experiments conducted with different wheat fertilization regimes (Schmalenberger et al., Environ Microbiol. June 2008;10(6):1486-1500). have demonstrated that arylsulphonates are important in the nutrition of desulphonating bacteria, and that the distribution of genera and species of desulphonating bacteria present in the soil for the growth of a plant vary greatly depending on the fertilisation regime used and the level of inorganic sulphate present in the fertiliser, although it is noteworthy that several strains of desulphonating bacteria isolated were of the genera *Variovorax* and *Polaromonas.*

Sulphate mobilisation of aromatic and aliphatic sulphonates is catalysed by a monooxygenase-type enzyme complex encoded by the *ssu* cluster. For the desulphonation of aromatic compounds such as arylsulphonates, ssu must be complemented by the asfRABC cluster, which comprises the asfA enxyme. Said enzyme is not only important because of its activity, but also because it is used as a marker for the sulphonatase activity of several soil bacteria such as *Variovorax, Polaromonas, Acidovorax and Rhodococcus,* and also in the phylogeny studies of bacteria capable of mobilising aromatic sulfonates, studies in which the analysis of 16S rRNA is also common. For instance, a partial sequence of *Variovorax sp.* DC2a-35 gene for 16S rRNA can be found disclosed in Database EM_STD [Online] 2 July 2010, "Variovorax sp. DC2a-35 gene for 16S rRNA, partial sequence", Database accession no. AB552859.

It has been proposed to complement or replace 16S rRNA analysis with the housekeeping gene *rpoB,* which encodes a protein (the beta subunit of RNA-directed RNA polymerase) and of which there is only one copy in each genome (Vos et al., PLOS ONE 7(2): e30600, https://doi.org/10.1371/journal.pone.0030600). These studies have expanded the diversity of beta-proteobacteria, the class of Gram-negative bacteria to which the genera *Variovorax* and *Acidovorax* belong, as well as *Thiobacillus,* a genus from which many members have disappeared and been reclassified into other genera due to genetic studies.

In fact, the *Comamonadaceae* family was a family created for organism belonging to the same rRNA complex as *Acidovorax* (Willems et al., International Journal of Systematic and Evolutionary Microbiology, vol 41(3):445-450), which included the genera *Comamonas, Acidovorax, Hydrogenophaga, Xylophilus,* and *Variovorax,* as well as some pathogenic *Pseudomonas* and *Aquaspirillium* species, that are closely related genotypically, although they are quite diverse phenotypically. With the creation of the family, the genus *Variovorax* was also created, with the organism formerly known as *Alcaligenes paradoxus* being transferred into this genus and renamed *Variovorax paradoxus.* Several other species of the same genus are now at present, such as: *V. boronicumulans, V. beijingensis, V. defluvii, V. dokdonensis, V. ginsengisoli, V. gossypii, V. guangxiensis, V. humicola, V. paradoxus, V. rhizosphaerae, V. robiniae, V. soli, V. ureilyticus.*

The complete genome sequence of one strain of *Variovorax paradoxus,* strain S110, and an extensive metabolic analysis of this strain can be found published (Han et al., Journal of Bacteriology 2011, 193(5):1183-1190). Both this strain and other species of the same genus are considered plant growth-promoting rhizobacteria that are good candidates for agrobiotechnological applications which include remediating degraded soils and increasing crop production, such as biofertiliser and biopesticide. It is also presented as a model of possible bacterial symbiosis with other microorganisms, being a common symbiont in the rhizosphere, capable of protecting plants by acting on toxic pollutants, promoting stress tolerance and disease resistance, and helping in the absorption of various nutrients. Among the compounds that it can metabolise include alkyl/arylsulphonates, as well as petroleum-associated sulphur metabolites. Its genome contains an alkane monooxygenase gene ssuD (which enables its activity on alkylsulphonates) and another oxidoreductase gene asfA (which allows it to metabolise arylsulphonates). During the metabolism of sulphur compounds, sulphite is generated, which can be assimilated by certain associated microorganisms or reduced to hydrogen sulphide. Sulphonate desulphurisation is a critical factor in the growth-promoting effect of *V. paradoxus,* with the genus *Variovorax* being mentioned in the article as a key player in the mineralisation of carbon-bound sulphur, contributing to the cycling of sulphur between organic and inorganic forms in soil.

The capabilities of *Variovorax paradoxus* in catabolism of sulphur compounds was reviewed by Satola *et al.* (Satola B. et al., "Metabolic characteristics of the species", Applied Microbiology and Biotechnology, vol. 97, no. 2, 29 November 2012, pages 541-560) mentioning its possible use in bioremediation, in plant growth stimulation and even in the degradation of toxic and/or complex chemical compounds. The review describes pathways of transformation of several sulphur compounds where *Variovorax paradoxus* can act, such as sulpholane, 3-sulpholene, 2-mercaptosuccinic acid, 3,3'-thiodipropionic acid, aromatic sulfonates, alkanesulfonates, amino acids and other sulphur sources.

US7329532B2 discloses methods of remediating sulphur-containing pollutants, the method comprising introducing a hydrocarbon to the sulphur-containing pollutant to stimulate growth of hydrocarbon-utilizing bacteria. *Variovorax* is mentioned as one of the possible hydrocarbon-utilizing bacteria that can be used while carrying out the method.

Wübbeler *et al*. (Wübbeler J. H. et al., "The genome of Variovorax paradoxus strain TBEA6 provides new understandings far the catabolism of 3,3'-thiodipropionic acid and hence the production of polythioesters", Journal of Biotechnology, vol. 209, 11 June 2015, pages 85-95) discloses the genome sequence of *Variovorax paradoxus* strain TBEA6, e.g. addressing utilization, conversion and detoxification of sulphite.

Bacteria of the genus *Variovorax* in general, and some specific strains in particular, have been proposed for various agrobiotechnological applications, sometimes in association with other microorganisms. In that sense, for example, document WO2020170244A1, which describes growth-promoting microorganisms and compositions containing them, as well as use thereof for promoting crop health, growth, and yield, or for enhancing traits such as yield, insect control, disease, drought or herbicide resistance, mentions a bacterium of the genus *Variovorax* as a possible microorganism to be used for these purposes. In particular, WO2020170244A1 discloses *Variovorax paradoxus* strain S3167, its 16S ribosomal RNA v1v9 region as SEQ ID NO:8 and its application in plant growth stimulation.

Other documents also disclose information about DNA sequences of *Variovorax paradoxus* strains. For instance, in the Database GenBank [Online], on 2 May 2023, "DNA-directed RNA polymerase subunit beta [Variovorax paradoxus]", Database accession no. WGT62599, it is disclosed DNA-directed RNA polymerase subunit beta from *Variovorax paradoxus* strain MGMM5, referring to GenBank:CP123990.1 (genome sequence of *Variovorax paradoxus* strain MGMM5) for its encoding nucleotide sequence.

In Dutta et al. ("Complete Genome Sequence of Variovorax paradoxus JBCE486, which Alleviates the Salt Stress of the Endemic Plant Ulleung-Sanrnaneul", MICROBIOLOGY RESOURCE ANNOUNCEMENTS, vol. 12, no. 1, 1 December 2022, pages 1-2) GenBank accession number CP102931 discloses a genome sequence of *Variovorax paradoxus* strain JBCW486, which strain is involved in plant growth.

Document CN110079471A, in turn, describes the *V. paradoxus* A06 strain with accession number CTCC No.: M 2018693 and growth-promoting effect, which improves yield, fertiliser effectiveness, and plant resistance. The study is conducted on the rhizosphere of legumes, in which hydrogen is introduced for the isolated culture of hydrogen-oxidising bacteria. The taxonomic study of bacteria includes the identification of 16S rDNA.

Document CN102747019B, in turn, describes a *Variovorax sp* strain, deposited under number CGMCC No. 5623, that can fix nitrogen and grow using insoluble phosphate as a phosphorus source, so it improves the degree of utilisation of fertilisers and facilitates the absorption thereof and root development. Its good growth promoting effect on peanuts is mentioned.

The specific use of *Variovorax strains* as fertilisers, or in combination with fertilisers, has also been disclosed in different documents. Document CN107759362A describes a fertiliser made up by an organic part (including, among others, Chinese medicine residues, humic acid, and diatoms), an inorganic part (with potassium nitrate, urea, potassium dihydrogen phosphate, potassium sulphate, and others) and a functional bacterium, which can be, among others, of the genus *Variovorax.*

In other documents, the bacteria themselves are the main component of the fertiliser, which is defined as microbial fertiliser. This is the case of document CN102603371, in which the proposed fertiliser must contain hydrogen-oxidising bacteria (hydroxide bacteria) and can be the fermentation liquor thereof, being able to also contain peat moss; the bacteria can be of the genera *Flavobacterium, Pseudomonas, Alcaligenes,* or *Burkholderia,* or mixtures thereof, with *Variovorax paradoxus* being mentioned as one of the possible bacteria to be used.

Along the same lines, document KR20180024795A describes a natural fertiliser comprising natural microorganisms as an effective ingredient and an additive capable of improving the effect of the microorganisms, maximising their growth-promoting effect, with guano and a surfactant being mentioned as possible additional ingredients. *Variovorax* is mentioned among the possible effective microorganisms.

Document RU2735429C1 also relates to bacterial fertilisers, which have a stimulating effect on plant growth under unfavourable conditions, comprising bacterial cells and nutrient medium residues, the bacterial component being a mixture of *Pseudomonas oryzihabitans* B3S (RCAM accession number 04752) and *Variovorax paradoxus* AV-10 (RCAM accession number 04753).

The association of *Variovorax* with other microorganisms to impart beneficial properties to target plants, especially plants from which crops are obtained, can be found described in several other publications. Document WO2017127535A1, for example, describes a microbial consortium that can be made up of at least two bacteria (one of which can be *Variovorax ginsengisoli),* and that can be used as a plant growth medium, or applied as a seed coating, influencing possible traits of interest such as modulated disease resistance, drought tolerance, heat tolerance, cold tolerance, salinity tolerance, metal tolerance, herbicide tolerance, chemical tolerance, improved water use efficiency, improved nitrogen use, pest and pathogen resistance, and several other traits of agronomic interest.

Reduction of pathogens in soil has also been disclosed for bacteria of the genus *Variovorax* in general, whole or lysed, together with intracellular components of rhizosphere-dwelling yeasts, as can be seen in document WO2012037352A2, in which the described compositions may also contain fertilisers.

Soil conditioning, either as a result of degradation due to excessive use of fertilisers or to reduce unwanted components, is also one of the properties for which the use of bacteria of the genus *Variovorax* has been suggested. For example, document CN115340420A describes a soil conditioner to reduce heavy metal content in crops, which conditioner is also a fertiliser containing as main elements waste biogas and livestock and poultry excrements, ammonium nitrate, and various sulphates, as well as bacteria, specifically *Bacillus mecilaginosus* and *Variovorax.* Document CN111154691A describes a Variovorax strain capable of degrading chitin and cellulose, the strain with CGMCC accession number 116908, which is a strain that can also convert insoluble phosphate into soluble phosphate in a targeted manner, improving the quality of soils degraded by excessive use of fertilisers, also improving the immunocompetence of plants.

In addition to their use as a fertiliser, or in combination with fertilisers, bacteria of the genus *Variovorax* have also been described as plant biostimulants or as components of microorganism stimulants with properties of interest, as can be found in document WO2022097152A1, which describes the *Variovorax* strain STS16 which appears to promote the degradation of the herbicides Linuron or Diuron.

Plant biostimulants are a relatively new product category, the definition and regulations for use of which are not yet fully developed. It is considered that plant biostimulants are compounds or microorganisms, as well as the compositions containing them, which help plants to improve and regulate their physiological and biochemical processes, stimulating plant nutrition processes independently of the nutrient content of the composition of which they are part (which differentiates them from fertilisers), generally based on two fundamental aspects: achieving a product based on a complex matrix and where the importance lies in the balance of substances supplied, and also that the active part of said product is a catalyst, substrate, or reagent of a metabolic reaction.

Therefore, a biostimulant seeks to shift the natural biochemical reactions of plants towards one or more traits that are favoured, successfully improving some of the following aspects: a) improvement in nutrient use efficiency; b) improved abiotic stress tolerance; c) attainment of crop quality characteristics.

Thus, biostimulants are not used to replace fertilisers, but they can be used together to achieve greater and better plant growth, since they provide additional protection against different types of stresses, use fertiliser nutrients more efficiently, and improve the absorption thereof.

Biostimulants can help to address inefficiencies in the field that persist today despite improved production practices. These products provide higher yields and quality, so they help farmers to produce more with less.

Each biostimulant may be formulated to cause different effects on different types of crops. They may have diverse specific uses according to the needs at any given time:
- Increasing crop tolerance to overcome abiotic stresses.
- Facilitating nutrient assimilation, translocation, and use.
- Improving plant metabolism efficiency to induce increased yield and improved crop quality.
- Improving quality attributes: increase in sugars, colour, crop quality, size, etc.
- Improving soil fertility; especially by means of enhancing the development of soil microorganisms.
- Achieving more efficient use of water.

Agricultural biostimulants therefore help to address some of the most important challenges facing world agriculture in the coming years. One notable example is feeding a growing population that requires increased crop yields, which can be enhanced by biostimulants. Extreme temperatures, water shortage, salinity, and other types of climate change-related stresses, require resilient crops to optimise yields. Along these lines, agricultural biostimulants increase plant tolerance to adverse effects of abiotic stresses, helping to protect and improve soil health, promoting the development of beneficial soil microorganisms. Healthy soil retains water more effectively and is more resistant to erosion. Furthermore, biostimulation minimises the use of chemicals because, by strengthening the defenses of the plant, it is healthier and stronger to cope with pests and diseases. It saves money and avoids waste.

Moreover, agricultural biostimulants can improve quality parameters of fruits and vegetables. Higher quality means higher profits for farmers and healthier and more nutritious food for consumers.

Generally, it is considered that biostimulants can be grouped into the following categories:
- Humic and fulvic acids (natural constituents of the top layer of soil, humus, resulting from the decomposition of living things, with humic acids being the substances extracted from soil humus which coagulate when treated with a strong base and fulvic acids the part that remains soluble, while the alkali-insoluble part of humus is called humin).
- Amino acids and peptide mixtures obtained from protein hydrolysis, as well as other nitrogenous substances such as betaines, polyamines, and non-protein amino acids
- Algae and plant extracts or purified compounds such as laminarin polysaccharides, alginate, and carrageenans.
- Chitosans (in polymeric or oligomeric form) and other biopolymers.
- Inorganic compounds (such as aluminium, cobalt, sodium, selenium, or silicon, present both in soil and in plants as different inorganic salts and as insoluble forms).
- Beneficial fungi, such as, for example, mycorrhizal fungi
- Beneficial bacteria.

Within the above classification, bacteria interact with plants in all possible ways. As with fungi, this interaction can range from parasitism to mutualism. Bacterial niches extend from the soil to the interior of plant cells, with intermediate locations such as the rhizosphere. These associations may be permanent or temporary, and some are transmitted through seed. They have a wide range of influence on the plant including biogeochemical cycles, nutrient supply, increased nutrient use efficiency, induction of disease resistance, improved tolerance to abiotic and biotic stresses, and even modulation of plant morphogenesis.

Currently, the new regulations on fertilisers and biostimulants for use in the European Union contemplate a section on beneficial soil microorganisms. The current list is made up of: *Azotobacter spp*.; mycorrhizal fungi; *Rhizobium spp,* and *Azospirillum spp.*

As this is such a new and innovative sector (until a year ago a definition of biostimulant was not recognised in the European Union), this list still does not include any sulphur-oxidising bacteria or ferrooxidans of the genus *Acidithiobacillus* or other genera related to sulphur or phosphate-related functions in the soil, despite the fact that they are fundamental microorganisms in the transformation of sulphate- and phosphate-type nutrients that are locked in the soil.

Although said microorganisms are not represented as such, the regulations contemplate the possibility of including new organisms once they have been characterised and shown to be effective for the stated purposes. For this reason, it is of particular interest to be able to introduce into the agricultural market another tool for sustainable, ecological use, with proven effectiveness and benefits not only for crops but also for soils.

The use of natural, non-genetically modified microorganisms requires a scientific investment in the process of searching, fermenting, and fine-tuning processes, which is probably equivalent to the investment required in the search for genetically modified microorganisms or other synthetic fungicides. However, the use of these microorganisms has the enormous advantage that they can perform functions that are currently unattainable by the latest recombinant technology, CRISPR-Cas, due to the intervention of complex operation and regulation gene systems. Furthermore, since they are natural soil microorganisms, there would be no regulatory barriers to their use in Europe, which barriers do exist for all genetically modified organisms.

Based on the foregoing, it would be interesting to have a naturally occurring microorganism capable of promoting plant growth and that could be used as a biostimulant. In particular, given the existing need to control the sulphur content of arable soils and, in the event of a deficiency of this element, establish the necessary sulphur addition strategies in order to move towards sufficient food production, it would be interesting for said microorganism to facilitate the use of elemental sulphur by plants, whether it is found naturally in the soil or whether it is supplied to the soil in the form of elemental sulphur particles which, as discussed above, present a series of advantages when added to the soil as such. Preferably, said microorganism must also exhibit other properties sought after in biostimulants.

The present invention provides a solution to that problem.

### Description of the Invention

The present invention provides a bacterium of the genus *Variovorax* capable of oxidising elemental sulphur (S⁰), giving rise to sulphate (SO₄²⁻), the main form of sulphur that can be absorbed by plant roots. Therefore, since it is present in the soil in which a plant grows, especially in the rhizosphere, it helps to meet the plant's needs for sulphur, increasing its availability for said plant, such that the plant can use it as nutrient, also favouring the absorption and utilisation of possible added fertilisers, giving rise to a good growth-promoting effect of the plant and increasing crop yield. In that sense, it is a plant growth-promoting bacterium.

It can be considered that the present invention is an alternative to the use of thiobacteria, wherein the bacterium that is used is a plant growth-promoting bacterium which is part of the microorganisms naturally present in the rhizosphere of plants, for which strains various effects that can be considered biostimulant have been described.

The invention is based on the discovery of a *Variovorax* strain isolated from nature which, as can be seen below in the section of Examples, exhibits this property. Specifically, it is the AFSI 2.2 strain deposited in the Colección Española de Cultivos Tipo (Spanish Type Culture Collection) (CECT) (Edificio 3 CUE, Parc Cientific Universitat de Valencia, Catedrático Agustin Escardino, 9 - 46980 Paterna, Valencia, Spain) on 25/10/2022 under accession number CECT 30738, the depositor being Azufrera y Fertilizantes Pallarés, SAU.

Thus, in a first aspect, the present invention relates to a bacterium of the genus *Variovorax,* characterised in that it is capable of transforming elemental sulphur into sulphate, which is the bacterium deposited in the Colección Española de Cultivos Tipo (Spanish Type Culture Collection) (CECT) under number CECT 30738.

This strain has been obtained from a study according to Royal Decree 124/2017, authorised by the General Directorate of Biodiversity, Forests, and Desertification of the Ministry for Ecological Transition and Demographic Challenge under number ESNC86, in which the authorisation for accessing Spanish genetic resources originating from wild taxa was granted in order to obtain native microorganisms from Spanish soils capable of solubilising soil nutrients that are not available for plants, for the development of solutions that allow avoiding chemical fertilisation with soluble nutrients that may leach out, improving the effectiveness of fertilisers. Specifically, the AFSI 2.2 strain was isolated from the farm where the facilities of Azufrera y Fertilizantes Pallarés SAU (AFEPASA) are located in Constanti, in the province of Tarragona.

The DNA amplification and sequencing test giving rise to the 16S rRNA (SEQ ID NO:1) and the comparison of the sequence with 16S ribosomal RNA sequences of other bacteria and archaea indicated that the bacterium is of the genus *Variovorax,* which was confirmed when the search was conducted by comparing the mentioned sequence with nucleotide sequences in general or when the search was conducted with a fragment of the *rpoB* gene also identified in the bacterium, as described in detail below.

The discovery of this strain, with the indicated characteristic, was surprising because, as set forth in the preceding section, strains of the genus *Variovorax* capable of oxidising elemental sulphur giving rise to sulphate were unknown. Until now, the main activity on sulphur compounds known for bacteria of this genus was the release of sulphate from organic sulphur, alkylsulphonate, and/or arylsulphonate compounds, with the *AsfA* gene, encoding the enzyme AsfA which participates in the transformation of arylsulphonates, being one of those commonly used for taxonomic studies relating to bacteria of this genus.

However, the tests performed show that one of the strains isolated within the framework of the mentioned study, AFSI 2.2, is capable of oxidising sulphur, giving rise to sulphate.

Furthermore, the tests performed with different horticultural plants (with horticulture being understood as the activity intended for the production of vegetables in both large and small farms, where vegetables include: legumes and greens, fruits, flowers, and aromatic herbs) and with a cereal, wheat, shows a biostimulant capacity on plant growth when added to irrigation water, with the increase generally being greater when it is added together with elemental sulphur.

As mentioned above, unlike direct addition in the form of sulphates, the addition of elemental sulphur to mitigate sulphur deficiency in soils has several advantages, among others, the prevention of losses by lixiviation as a result of not being water-soluble. The bacteria of the present invention, when added to a soil, directly to the root ball of the plant, or in the nutrient solution with which hydroponically cultivated plants are irrigated, enable or collaborate in ensuring that there are sufficient microorganisms in that soil, or in the conditions of cultivation of the plant, to transform said sulphur into the form that can be assimilated by the plants, i.e., sulphate. This transformation is progressive, occurring throughout the vegetative cycle, and is also, to a certain extent, controlled since, with the reaction being chemically balanced, whenever there is a sulphate deficiency (which is favoured by the absorption of sulphate by the roots of the plants in order to meet their metabolic needs), the reaction will shift towards the production of a higher amount of said anion.

In addition to the direct nutritional value entailed by the supply of formed sulphate, this process gives rise to an indirect nutritional value such as soil conditioning, favouring the assimilation of microelements in an optimal pH range. Furthermore, along with the notable reduction of pH and conductivity, sulphate ion serves as a vehicle so that vegetables can incorporate microelements, being absorbed in the form of sulphates (iron, copper, zinc sulphates, etc.). Efficiency in the use of other essential nutrients for the plants, particularly nitrogen and phosphorus, is also improved, since sulphate formation contributes to the solubilisation of insoluble phosphorus salts present in soil, due to the action of the sulphuric acid formed, which will be in equilibrium with sulphate. With respect to nitrogen, furthermore, as set forth above, it has been demonstrated that an equilibrium between sulphur and nitrogen is essential for improved nitrogen assimilation, preventing typical losses by evaporation.

Therefore, another aspect of the invention relates to a composition comprising the bacterium of the invention. Said composition may comprise, among others, at least one additional biostimulant. Furthermore, optionally, the composition may also comprise elemental sulphur.

The properties of the strain, giving rise to an increased yield of the plants cultivated in a substrate to which the bacterium of the present invention has been added, allow proposing, as part of the invention, the use of the bacterium of the invention, or of a composition comprising the same, to promote plant growth and/or to act as a biostimulant for the plants. Due to the properties of the bacterium of the present invention, these effects will be favoured in the presence of sulphur, so it is preferred that elemental sulphur is present in the soil in which the plant grows or in the nutrient solution delivered to a hydroponically cultivated plant.

The invention can be particularly useful for cultivating plants with particularly high sulphur requirements, as is often the case with plants of the *Brassicaceae* family, such as the plants of the genus *Brassica,* such as the different varieties of the species *Brassica oleracea* (for example, cabbage (*Brassica oleracea var. capitata*)*,* broccoli (*Brassica oleracea var. italica* Plenck), or cauliflower (*Brassica oleracea var. botrytis*))*.*

It is also worth noting that, being a process in which oxygen is consumed, the transformation of elemental sulphur into sulphate contributes to making soils more porous and favouring their oxygenation, to which the larger steric volume of the sulphate ion, which is larger than that of elemental sulphur, also contributes, thus widening the space occupied by the latter. Furthermore, this process favours the conditioning of alkaline, limestone, and saline soils due to the notable reduction in pH and conductivity caused therein.

Therefore, the invention can also be useful for the modification, conditioning, and/or recovery of soils, enabling the enrichment of a soil with sulphur salts, or also for the modification of properties such as pH or conductivity or porosity. In that sense, another aspect of the present invention relates to a method for the modification and/or restoration of soil comprising at least one step of adding a bacterium of the present invention or a composition comprising a bacterium of the present invention to the soil. The modification caused in the soil can be, for example, enrichment with sulphur salts, solubilisation of phosphorus salts, reduction of pH, increase of conductivity, increase of porosity, or mixtures of the above.

Possible ways of carrying out the invention are explained in greater detail below.

### Brief Description of the Drawings

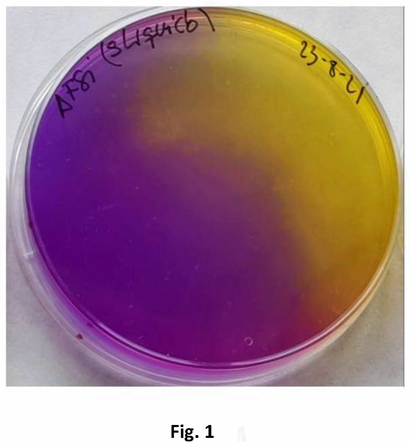

Fig. 1 shows a plate with bromocresol purple in which the right-side area can be seen in a lighter colour (yellow in the original) due to the change of the original purple colour as a result of the action of the bacteria growing in said area.

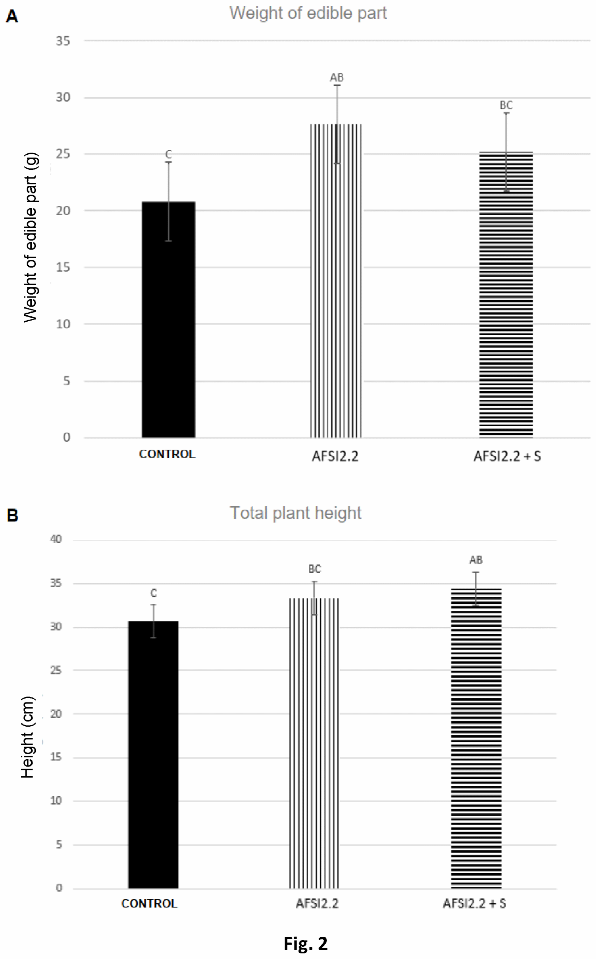

Fig. 2 shows the results, related to the weight of edible parts (panel A) and the height of the plants (panel B), obtained in a greenhouse broccoli test in control plants (leftmost bar on each graph, filled in black), plants treated with the strain of the invention (middle bar, filled with vertical lines), and plants treated with the strain and elemental sulphur (rightmost bar on each graph, filled with horizontal lines). Each bar represents the mean value corresponding to 8 plants. Standard deviation is also depicted. Different letters above the bars indicate statistically significant differences between treatment groups.

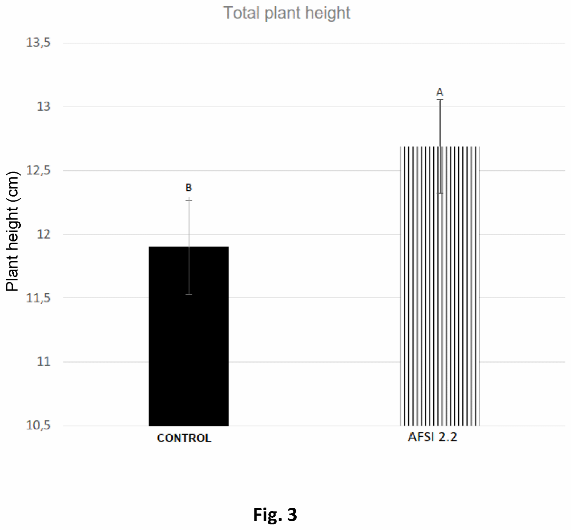

Fig. 3 shows the results, related to the height of the plants, obtained with cabbage in a greenhouse test in control plants (leftmost bar on each graph, filled in black) or plants treated with the strain of the invention (rightmost bar on each graph, filled with vertical lines, labelled "AFSI 2.2"). Each bar represents the mean value corresponding to 18 plants. Standard deviation is also depicted. Different letters above the bars indicate statistically significant differences between the two treatment groups.

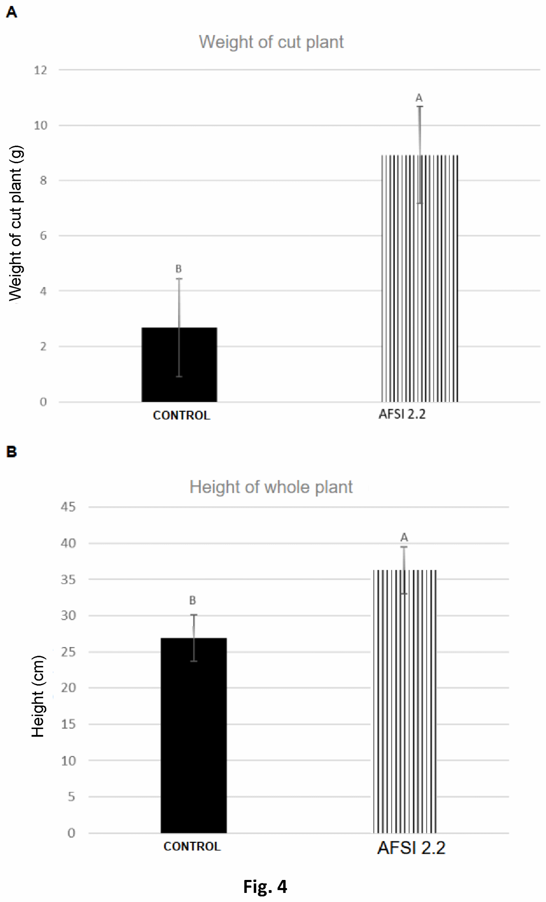

Fig. 4 shows the results, related to the weight of cut plants (panel A) and the height of whole plants (panel B), obtained with wheat in a greenhouse test, in control plants (leftmost bar on each graph) or plant treated with the strain of the invention (rightmost bar on each graph, labelled "AFSI 2.2"). Each bar represents the mean value corresponding to 12 plants. Standard deviation is also depicted. Different letters above the bars indicate statistically significant differences between the two treatment groups.

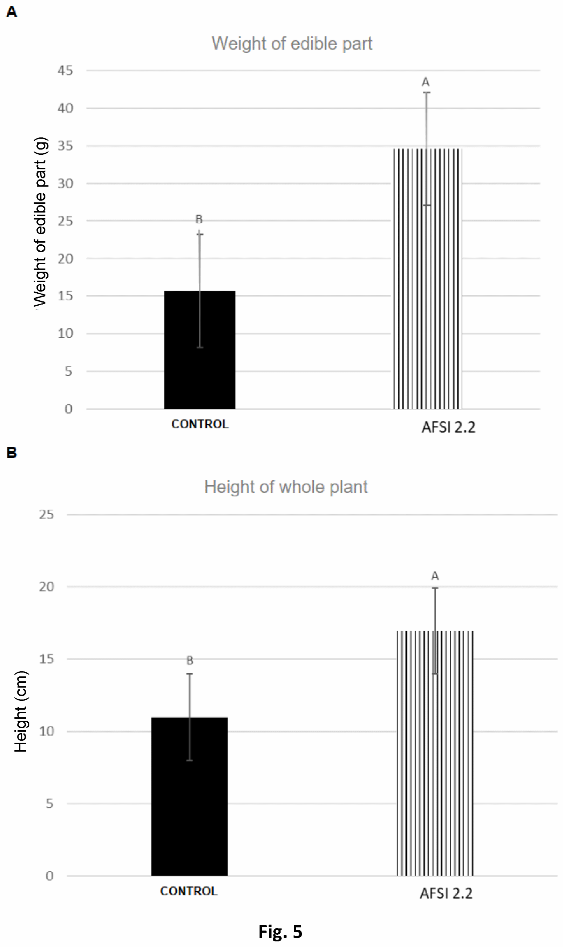

Fig. 5 shows the results, related to the weight of edible parts (panel A) and the height of whole plants (panel B), obtained with radish in a greenhouse test, in control plants (leftmost bar on each graph) or plants treated with the strain of the invention (rightmost bar on each graph, labelled "AFSI 2.2"). Each bar represents the mean value corresponding to 12 plants. Standard deviation is also depicted. Different letters above the bars indicate statistically significant differences between the two treatment groups.

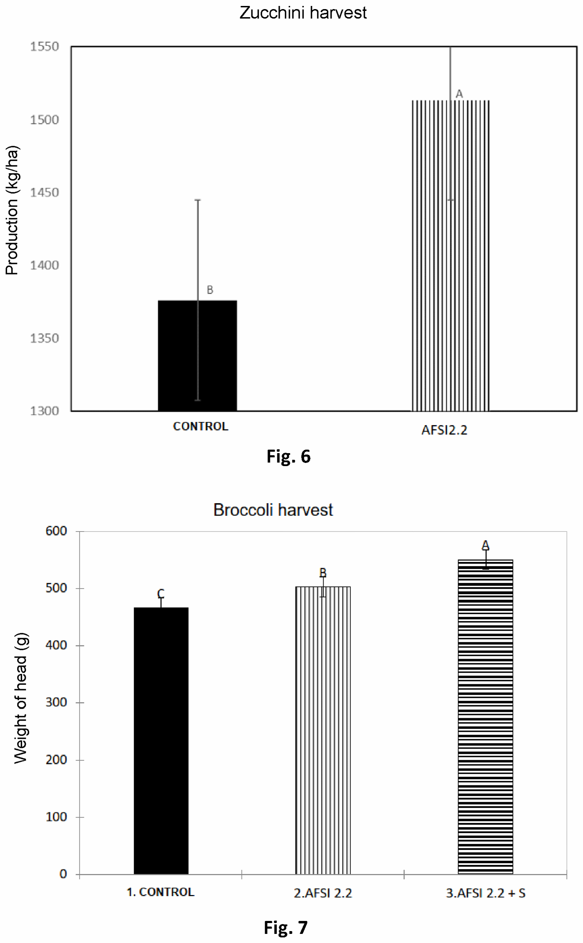

Fig. 6 shows the results obtained in the production of zucchini harvested after cultivating in the open air 40 control plants (leftmost bar on each graph) or 40 plants treated with the strain of the invention (rightmost bar on each graph, labelled "AFSI 2.2"). Standard deviation is also depicted. Different letters above the bars indicate statistically significant differences between the two treatment groups.
Fig. 7 shows the results, related to the weight of edible parts, obtained in an open-air broccoli cultivation test, in control plants (leftmost bar of the graph, filled in black), plants treated with the strain of the invention (middle bar of the graph, filled with vertical lines and labelled "2.AFSI 2.2"), or plants treated with the strain and with sulphur supply (middle bar of the graph, filled with horizontal lines and labelled "3.AFSI 2.2 + S"). Each bar represents the mean value corresponding to 51 plants. Standard deviation is also depicted. Different letters above the bars indicate statistically significant differences between the two treatment groups.

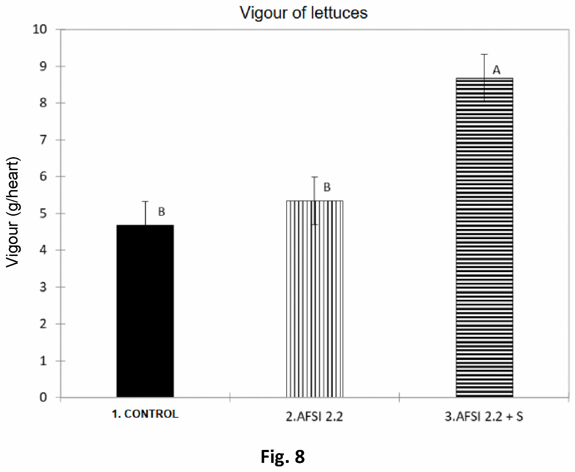

Fig. 8 shows the results, related to the vigour of the plants, obtained in an open-air lettuce cultivation test, in control plants (leftmost bar of the graph, filled in black), plants treated with the strain of the invention (middle bar of the graph, filled with vertical lines and labelled "2.AFSI 2.2"), or plants treated with the strain and with sulphur supply (middle bar of the graph, filled with horizontal lines and labelled "3.AFSI 2.2 + S"). Each bar represents the mean value corresponding to 12 plants. Standard deviation is also depicted. Different letters above the bars indicate statistically significant differences between the two treatment groups.

### Preferred Embodiments of the Invention

As discussed above, the invention relates to a bacterium of the genus *Variovorax,* characterised in that it is capable of transforming elemental sulphur into sulphate (SO₄²⁻), which is the bacterium deposited in the Colección Española de Cultivos Tipo (Spanish Type Culture Collection) (CECT) under number CECT 30738. The bacterium also has the capacity to promote plant growth.

Preferably, it is a bacterium which:
a) comprises in its genome a DNA fragment with the sequence represented by SEQ ID NO:2; and/or
b) comprises in its genome a DNA fragment with the sequence represented by SEQ ID NO:3;
   and/or
c) comprises in its genome a DNA fragment with the sequence represented by SEQ ID NO:4,
   and/or
d) comprises in its genome a DNA fragment with the sequence represented by SEQ ID NO:5.

As shown in Example 1, the bacterium also comprises in its genome a DNA fragment with the sequence of SEQ ID NO:1.

The DNA fragment with the sequence of SEQ ID NO:2, corresponding to a fragment of the rpoB gene, has a percentage of sequence identity of 96.58% with a fragment of the complete sequence of Variovorax sp. RKNM96, with higher percentage of identity being found when performing a sequence comparison; therefore, the sequence of said fragment is enough by itself to characterise the bacterium of the present invention and to distinguish it from any other. In turn, the sequence of SEQ ID NO:3, also corresponding to the rpoB gene but is much broader, comprising the complete coding sequence of the product of said gene (protein of SEQ ID NO:6), also characterises the bacterium of the present invention. The sequence of SEQ ID NO:4 and/or the sequence of SEQ ID NO:5 which, together, seem to represent the complete genome of the bacterium also characterise the bacterium and represent an alternative for same, so any of the sequences, or the verification of the presence of both, also allows identifying the bacterium of the present invention. In turn, the DNA fragment with the sequence of SEQ ID NO: 1 corresponds to the DNA of 16S ribosomal RNA of the bacterium of the present invention and characterises it in combination with any of the foregoing sequences, particularly complementing SEQ ID NO:2 or SEQ ID NO:3.

Any of the sequences SEQ ID NO:2, SEQ ID NO: 3, SEQ ID NO: 4, SEQ ID NO: 5, characteristic of the bacterium of the present invention and not found identically in another organism, can be used to identify the bacterium of the present invention, individually or in combination with the verification of the presence of combinations thereof. In this way, a method for identifying the presence of a bacterium of the invention in a sample or for identifying that a bacterium is the bacterium of the invention and not another one may include the step of verifying the presence of one of the sequences of SEQ ID NO:2, SEQ ID NO: 3, SEQ ID NO:4, or SEQ ID NO:5, or combinations thereof. Verification of the presence of one or more of said sequences could be combined, for greater certainty, with the verification of the presence of a DNA fragment with SEQ ID NO:1. To that end, the methodologies described below in Example 1 can be followed. Primers 27F 5' agagtttgatcctggctcag 3' (SEQ ID NO:9) and 1522R 5' aaggaggtgatccanccrca 3' (SEQ ID NO:10) can also be used for the DNA of the 16S rRNA and Univ_rpoB_ deg 5' ggytwygaagtncghgacgtdca 3' (SEQ ID NO:11) and Univ_rpoB_R_deg 5' tgacgytgcatgttbgmrcccatma 3' (SEQ ID NO:12) can be used for the rpoB gene for PCR amplification, or preferably massive or next-generation sequencing techniques such as Illumina^{®} techniques (San Diego, California, USA: https://www.illumina.com), or any other method known by those skilled in the art which allows identifying the presence of a DNA fragment with a known sequence in a sample.

In that sense, it must be taken into account that, as it is used herein, the expressions "with the sequence of" or "having the sequence of", referred to a DNA fragment or a DNA molecule and followed by "SEQ ID NO:", means that the DNA fragment or DNA molecule is represented by the nucleotide sequence identified by that "SEQ ID NO", that is, even though the DNA fragment or DNA molecule is or might be double-stranded, the nucleotide sequence identified by that "SEQ ID NO" is the nucleotide sequence of one of the strands, indicated from the 5' end to the 3' end, the sequence of the other strand being deducible from the rules of complementarity related to DNA molecules. The expression "a DNA fragment", in turn, does not necessarily refer to a DNA fragment split or separated from a larger molecule, but to a specific part or section of a DNA molecule.

The bacterium is specifically the strain isolated by the present inventors, in other words, the bacterium is the strain deposited in the Colección Española de Cultivos Tipo (Spanish Type Culture Collection) (CECT) under number CECT 30738.

The invention also relates to a composition comprising the bacterium of the invention. The concentration of the bacterium in said composition can be variable according to the plant to which the composition will be delivered and the characteristics of the soil in which the plant is growing, but value in the concentration range of 10⁶ - 10⁸ CFU/ml (colony forming units per millilitre), particularly the value of 10⁸ CFU/ml, can be taken as guide values.

The bacterium of the present invention has, by itself, biostimulant properties, favouring the growth and yield of cultivated plants when this bacterium is present in the growth soil or in the nutrient supply medium, by giving rise to the elemental sulphur-sulphate transformation reaction, which favours the absorption of sulphur in said soluble form by the bacteria. it fulfils the properties of a biostimulant since it stimulates a natural process, promoting plant growth and development, but without itself providing nutrients. However, the compositions of the present invention may also comprise one or more additional biostimulants. Examples of possible biostimulants that may be present in the compositions of the present invention are humic acids and/or fulvic acids, amino acids, peptides from protein hydrolysis, algae extracts, laminarin polysaccharides, alginate, carrageenan, polymeric chitosan, a chitosan oligomer, elemental aluminium, an aluminium salt, elemental cobalt, a cobalt salt, elemental sodium, a sodium salt, elemental selenium, a selenium salt, elemental silicon, a silicon salt, a mycorrhizal fungus, another biostimulant bacteria, or mixtures of the above.

Fulvic acids, for example, favour plant growth and development, so they may be of interest when that is the trait to be favoured, also having the advantage of being soluble in alkaline solutions. In turn, humic acids are often applied to improve soil structure, so they can be interesting components of the compositions of the present invention, particularly when the composition is used in a method of the present invention for the modification and/or restoration of soil. A mixture thereof in the same composition can also be used, which is a preferred embodiment of the compositions of the present invention, particularly when elemental sulphur is also present in the same composition. Possible preferred embodiments are also those which incorporate other additional biostimulants, such as any of those mentioned in the preceding paragraph.

Other biostimulants, such as those from the group of amino acids, peptides from protein hydrolysis, algae extracts (such as, for example, of the genus *Chlorella*)*,* laminarin polysaccharides, alginate, carrageenan, polymeric chitosan and/or chitosan oligomers, may be suitable when enrichment of the soil with organic matter is sought, in addition to the biostimulant effect. As in the case of the preceding biostimulants, the presence of one or more biostimulants in a composition of the present invention is compatible with the presence of any other biostimulant or another additional component, such as elemental sulphur or another inorganic sulphur compound.

In terms of the possible additional biostimulant microorganisms, thiobacteria, which can work together with the bacteria of the invention in the oxidation of elemental sulphur to sulphate, are particularly suitable for the purposes of the invention. Some of them have additional properties suitable for the purposes of the present invention such as, for example, *Acidithiobacillus ferrooxidans* (which metabolises iron and sulphur, producing sulphate), *Acidithiobacillus thiooxidans* (which metabolises elemental sulphur, producing sulphate). Root-associated mycorrhizal fungi may also be suitable, among which the fungi forming arbuscular mycorrhizae, belonging to the Glomeromycetes class are commonly used as biostimulants.

Preferably, regardless of whether or not one or more additional biostimulants are present, a composition of the present invention will also comprise elemental sulphur, but it is not essential, given that, with elemental sulphur existing in the soil, the bacteria of the invention will facilitate its oxidation to sulphate, without needing an additional supply. The sulphur can be, for example, in the form of granules, powder, or pellets.

The compositions of the present invention may comprise any other additional components, regardless of whether or not additional biostimulants and/or elemental sulphur are present, especially if they are common components of compositions for agricultural or agronomic use. Possible additional components of interest may be, for example, plant protection products, one or more fertilisers, or one or more inorganic sulphur compounds other than elemental sulphur, such as sulphur trioxide (SO₃), or even some sulphate salt since, although the bacteria of the invention favour the oxidation of elemental sulphur into sulphate ion, the invention is not incompatible with the presence of sulphates or other sulphur compounds, in the same composition as the bacteria of the present invention.

It is preferred for the bacteria of the present invention to be delivered with the irrigation water for the plant or, in the case of hydroponic crops, with the solution with which nutrients are delivered to the plants, so it is preferred for the compositions of the present invention to be aqueous compositions, but any other compositions are equally comprised within the scope of the present invention provided that they comprise the bacterium of the present invention.

Solid compositions comprising the bacterium of the present invention, that can be supplied to the plants by placing them beside or next to the plants, on the soil surface, or even by applying them on a layer of the soil, preferably on the upper humus layer, are also comprised within the scope of the present invention. The solid compositions will be delivered to the soil as non-particulate solids or preferably in the form of particles, which can be in the form of granules of different sizes, or in the form of pellet or powder. Granule is understood to mean a small aggregate, usually in the form of a dry sphere, with a mean size of between 0.05 and 7 mm. In turn, a powder is formed by more or less fine solid particles, usually with a mean size of between 30 and 500 µm and obtained by crystallisation, drying, precipitation, or grinding. In contrast, a pellet is an aggregate in the form of a cylinder, usually manufactured by compression and extrusion and with a mean size of between 0.5 and 3 cm.

When the bacteria of the present invention are delivered in irrigation water, or when the composition of the present invention, in any of the possible embodiments related to the possible components thereof, is in the form of irrigation water, as well as also when a bacterium of the invention or a composition of the invention is used as a biostimulant or as part of a method of the invention for the modification, conditioning, restoration, or bioremediation of a soil, irrigation can take place through any of the common methodologies: surface or gravity irrigation (including furrow irrigation, border or flowerbed irrigation, strip irrigation, spreading irrigation using sluices, terrace irrigation, irrigation by ancestral water channels located on a hillside with small outlets, and pond irrigation), sprinkling, micro-sprinkling, drip irrigation, jet irrigation, hydroponic irrigation, misting and spraying (including modalities using drones with cameras and specific sensors for detecting the needs of each area and the state of vegetation), underground irrigation (for example, by humidifiers placed underneath the plant), etc. Even in specific cases, direct injection, for example, into the root ball, such as in some of the examples shown below, can be used.

The present invention is compatible with use thereof for promoting growth and/or acting as a biostimulant (promoting said characteristic or any other interesting characteristic, particularly the yield or a factor that influences the quality) of any plant. Horticultural plants or cereals, for example, those used in the examples described below are preferred: broccoli (*Brassica oleracea var. italica*)*,* cabbage (*Brassica oleracea var. capitata*)*,* radish (*Raphanus sativus,* also belonging to the *Brassicaceae* family), lettuce (*Lactuca sativa*)*,* zucchini (*Cucurbita pepo*)*,* or wheat as a cereal (*Triticum aestivum* or *Triticum vulgare,* as well as other species of wheat, such as *Triticum spelt, Triticum durum, Dicoccum wheat,* or even *Fagopyrum esculentum,* the wheat known as buckwheat, or also hybrid varieties). Plants from the *Brassicaceae* family in general, and of the genus *Brassica* in particular, as explained above, are also a possible preferred embodiment of the plant for use in the present invention.

Both in the use of the bacterium of the present invention or of a composition comprising same to promote plant growth and/or act as a plant biostimulant, and in the method of the present invention for the modification, conditioning, restoration, or bioremediation of a soil, the bacterium of the invention or the composition the invention can be delivered in a single dose (denominated "application" in the Examples section) or in more than one dose (application), separated in time; this last alternative is the one chosen in the examples presented below. As discussed above, elemental sulphur also may or may not be delivered. If elemental sulphur is also delivered, it can be delivered simultaneously with the bacterium of the present invention, preferably being part of the same composition, or it can be delivered in doses (applications) different from the doses of delivery (applications) of the bacterium of the present invention or of a composition of the present invention. Preferably, if delivered in different doses (applications), said doses (applications) will be separated in time. Preferably, if more than one dose (application) of elemental sulphur and more than one dose (application) of the bacterium of the present invention or of a composition of the present invention are delivered, the deliveries (also denominated "application" in the Examples section) will be in alternation, like in the last test of the examples of the specification. The time interval between one delivery (application) and another can be, for example, one week, such as in said test, or another different time interval that is deemed to be more suitable according to the specific conditions of the soil and/or the cultivated plants.

Therefore, it can be said that possible embodiments of the use of the present invention are those in which elemental sulphur is delivered to the plant. These embodiments comprise the alternatives of sulphur and bacteria being delivered together and simultaneously (for example, in the same composition) or of elemental sulphur being delivered separately in a different composition and separately in time, such that elemental sulphur is delivered to the plant in one or more doses (applications) different from the dose/doses of delivery (application/applications) of the bacterium of the present invention or a composition of the present invention which, in this case, will not comprise elemental sulphur. Thus, elemental sulphur and bacteria of the present invention can be delivered in the same composition or in different compositions and they can be delivered simultaneously or separate in time.

Similarly, in a possible embodiment, the method of the present invention may comprise one or more steps in which elemental sulphur is delivered to the soil. Possible embodiments thereof are those in which sulphur is delivered together with the bacterium of the present invention (which is compatible with supplying to the soil a composition of the present invention comprising elemental sulphur), or those in which they are delivered separately, preferably separated in time. A preferred embodiment of the method of the invention is an embodiment in which elemental sulphur is delivered in one or more steps (applications) different from the step/steps of delivering the bacterium of the present invention or a composition of the present invention lacking elemental sulphur. In said possible embodiment, if there is more than one step (application) of delivering elemental sulphur and more than one step (application) of delivering the bacterium of the present invention, it is preferred to alternate said steps (applications).

Possible embodiments of the use of the present invention or of the method thereof also comprised within the scope of the present invention are those in which there is more than one dose/steps (applications) of delivering the bacteria of the present invention and at least two of them are consecutive, without there being a need for a step of delivering sulphur between them, like in the first tests of Example 2 of the present specification. Likewise, there may be a single step of delivering sulphur and a single step of delivering bacteria regardless of whether or not sulphur is delivered.

Other possible combinations are also comprised in the scope of the present invention.

Examples which will illustrate the present invention and its possible embodiments in greater detail are included below.

### Examples

### Example 1. Isolation, selection, characterisation

### 1.1. Isolation of microorganisms

Within the framework of the study in accordance with Royal Decree 124/2017, authorised by the General Directorate of Biodiversity, Forests, and Desertification of the Ministry for Ecological Transition and Demographic Challenge under number ESNC86, wild soil samples were taken from the facilities of the applicant, Azufrera y Fertilizantes Pallarès SAU (abbreviated hereinafter as AFEPASA), located in the industrial park of Constanti, in the province of Tarragona, an area that is particularly rich in elemental sulphur.

In addition to starting from a soil very rich in sulphur, which in itself models the microbial populations, enhancing those involved in the sulphur cycle, a method known for years was carried out for the isolation, said method consisting of enriching the soil sample in a medium containing elemental sulphur to enhance the development of sulphur-oxidising bacterial communities and to be able to select them, such that the detection of sulphur-oxidising bacteria is facilitated.

In this way, to carry out the isolation, based on an approach of enriching the soil with sulphur-oxidising bacteria, aliquots of soil samples were suspended in water and kept under stirring for 2 h, after which dilutions of the soil samples up to 10⁻⁶ were prepared. From the last three dilutions, 100 microlitres were seeded in Erlenmeyer flasks. To that end, a series of Erlenmeyer flasks were set up with three different media, two containing elemental S:: liquid Twin Pack broth containing micronised elemental S with added dispersant and the same medium with added liquid S at 85% purity, and another liquid medium, Thiosulphate Medium:

**Sulphur medium: TWIN PACK broth**

| *Ingredients* | *g*/*litre* |
|---|---|
| Part A | |
| KH₂PO₄ | 3 g |
| MgSO₄·7 H₂O | 0.5 g |
| (NH₄)₂ SO₄ | 0.3 g |
| CaCl₂.2 H₂O | 0.250 g |
| FeCl₃.6 H₂O | 0.02 g |

| Part B | |
|---|---|
| Elemental sulphur | 10 g |
| final pH (25°C) | 4.8±0.2 |

**Thiosulphate Medium**

| *Ingredients* | *g*/*litre* |
|---|---|
| Na₂S₂O₃ | 5 g |
| KH₂PO₄ | 0.1 g |
| NaHCO₃ | 0.2 g |
| NH₄Cl | 0.1 g |

The Erlenmeyer were placed under constant stirring at 160 rpm and at a temperature of 28°C, thus being incubated for 25 days, after which one aliquot was extracted from each culture and inoculated again in another round of Erlenmeyer flasks with the same culture medium, incubating them for 25 days more in the same conditions.

Aliquots from these last cultures were seeded in Petri plates with solid thiosulphate culture medium, adding 1.5% agar and traces of bromocresol purple as an indicator, colonies which change the medium from purple colour to yellow colour (Fig. 1), due to the production of acid as a result of the oxidation of thiosulphate, which indicated that the isolated strain corresponded to sulphur-oxidising bacteria. This culture medium is typically used in the detection and isolation of sulphur-oxidising bacteria since, by using elemental sulphur or thiosulphate as an energy source, they produce sulphuric acid that changes the pH of the medium to acidic values which are detected by the indicator (bromocresol purple), according to the protocols and methods established by Veerender et al., 2014 Progressive Research 104-107; Reddy et al., 2018 Int J Curr Microbiol App Sci 7: 2639-2644.

Following the preceding method, the bacterial strain which was named AFSI 2.2 was selected as the bacterium capable of oxidising elemental sulphur and transforming it into sulphate.

It was verified that trypticase soy agar (TSA) CECT 20 can be used as a culture medium for the bacterium in aerobic conditions. Incubation time: 48 - 72 hours. Incubation temperature: 26°C ± 2°C. It is freeze- and lyophilisation-resistant.

### 1.2. Characterisation

Secugen S.L. (Madrid, Spain) was in charge of taxonomic classification. To that end, from a colony isolated from a culture plate, the total DNA of the bacterium was obtained by standard methods, using the QIAcube^{®} robot and the QIAamp^{®} DNA Blood Mini kit (Qiagen, Hilden, Germany) and, based on same, the gene giving rise to the 16S rRNA was amplified using universal primers 16SF1-M13-F17: 5' gtaaaacgacggccagtgastttgatcctggctyag 3' (SEQ ID NO:7) and 1492-M13-R19: 5' ggaaacagctatgaccatgcggttaccttgttacgactt 3' (SEQ ID NO:8) in the following PCR conditions: 15 minutes at 95°C: (30 seconds at 95°C + 40 seconds at 56°C + 1 minute at 72°C) x 35 + 8 minutes at 72°C + 4°, using Applied Biosystems TaqGold^{®} polymerase (Massachusetts, USA). Sequencing of the amplified fragment was carried out from both ends by means of Applied Biosystems BigDye 3.1 reagent (Massachusetts, USA), and subsequent capillary electrophoresis in an ABI 3730xl automatic sequencer (Thermo Fisher Scientific, Alcobendas, Madrid, Spain), obtaining the sequence represented by SEQ ID NO:1. The comparison of the sequence, by means of the basic local alignment search tool, BLAST (Altschul et al., Journal of Molecular Biology 215 (3): 403-410) of the National Center for Biotechnology Information, NCBI (accesible en: https://blast.ncbi.nim.nih.gov/Blast.cgi?PROGRAM=blastn&BLAST_SPEC=GeoBlast&PA GE_TYPE=BlastSearch) with the reference sequences existing in the database of 16S ribosomal RNA sequences of other bacteria and archaea ("16S ribosomal RNA sequences (Bacteria and Archaea)", accessible at: https://www.ncbi.nlm. nih.gov/nuccore?term=33175 %5BBioProject%5D+OR+33317 %5B BioProject%5D), indicated that the bacterium is of the genus *Variovorax.* The compared fragment exhibited an identity greater than 99.5% but less than 99.70% (specifically, 99.65%) with a strain of the *Variovorax paradoxus* species (specifically the strain NBRC 15149), and an identity greater than 98.5% with several other *Variovorax* strains. Comparison of SEQ ID NO:1, also with the BLASTN tool, with the nucleotide collection accessible in standard NCBI databases (which is a non-curated database, and therefore, with a lower degree of revision and control compared to the preceding one), without restrictions with respect to the possible encoded product, confirmed the attribution of the strain to the genus *Variovorax* since, of the first 24 sequences with respect to which a high percentage of identity was obtained (percentage ranging from 100.00% to 99.58%), only the three strains for which genus was not identified did not appear as members of the genus *Variovorax.* In particular, SEQ ID NO:1 has 100.00% nucleotide identity over 1441 positions in a common overlap (range (q:s): 1-1441:8-1448) with the partial sequence of Variovorax sp. DC2a-35 gene for 16S rRNA which can be found disclosed in Database EM_STD [Online] 2 July 2010, "Variovorax sp. DC2a-35 gene for 16S rRNA, partial sequence", Database accession no. AB552859. Moreover, SEQ ID NO:1 of the present application has 99.86% identity in nucleotides over 1441 positions in a common overlap (range (q:s): 1-1441:8-1448) with SEQ ID NO:8 of WO2020/170244A1, which represent 16S ribosomal RNA v1v9 region of Variovorax paradoxus strain S3167.

Subsequently, amplification of a partial sequence of the rpoB gene (SEQ ID NO:2) was carried out in IRNASA-CSIC with primers Univ_rpoB_F deg 5' ggytwygaagtncghgacgtdca 3' (SEQ ID NO:11) and Univ_rpoB_R_deg 5' tgacgytgcatgttbgmrcccatma 3' (SEQ ID NO:12) (Ogier et al., 2019 (BMC Microbiology (2019) 19:171 https://doi.org/10.1186/s12866-019-1546-z).

The sequence of SEQ ID NO:2 again confirmed the attribution of the strain to the genus *Variovorax.* Said sequence showed a higher percentage of identity with sequences corresponding to the *rpoB* gene of strains of the genus *Variovorax,* with the highest percentages being those corresponding to the *Variovorax sp.* RKNM96 strain (96.58%), *Variovorax sp.* PMC12 strain (96.32%), *Variovorax sp.* PDNC026 strain (96.32%), *Variovorax sp.* PAMC26660 strain (96.32%), and *Variovorax paradoxus* EPS strain (96.32%), and also with *Variovorax paradoxus* MGMM5 strain (95.88%) over 364 positions in a common overlap (range (q:s): 1-364:4460854-4461217) with regard to MGMM5 chromosome complete genome (length: 5810675) as disclosed in GenBank:CP123990.1, as well as with *Variovorax paradoxus* JBCE486 strain (95.88%) over 364 positions in a common overlap (range (q:s): 1-364:6047790-6047427) with regard to the chromosome of said strain, complete genome (length: 7294742) as disclosed in GenBank:CP102931. SEQ ID NO:3 has 96.99% nucleotide identity over 4125 positions in a common overlap (range (q:s): 1-4125: 4459174-4463298) with regard to *Variovorax paradoxus* MGMM5 chromosome complete genome (length: 5810675) as disclosed in GenBank:CP123990.1, and 96.19% nucleotide identity over 4121 positions in a common overlap (range (q:s): 1-4121: 6049470-6045350) with regard to *Variovorax paradoxus* JBCE486 chromosome complete genome (length: 7294742) as disclosed in GenBank:CP102931.

For genome sequencing, bacterial DNA was extracted with Qiagen MagAttract HMW DNA kit (Qiagen Iberia, Las Rozas, Spain). The preparation was enriched with Oxford Nanopore, ON, Short Fragment Eliminator reagent (Oxford, United Kingdom). DNA sequencing was performed with ON technology: libraries were generated with the SKQ-LSK114 kit and labelled by means of ligation with SQK-NBD114.96 barcoding, and sequencing was performed with the MinION equipment in a FLO-MIN114 vR10.4.1 flow cell at a sequencing speed of 400 bp/s, obtaining 560.4 Mb of sequence. Readings were reanalysed with super accurate quality and a mean quality of 20.4 was obtained. A duplex-type analysis was performed to obtain higher quality readings. Readings that do not contribute to duplex-type readings are selected and added to the duplex readings to perform assembly after eliminating readings with quality below 11. Quality analysis was performed with Nanoplot software version 1.36.2 (De Coster, W. (2018). NanoPlot. In GitHub repository. https://github.com/wdecoster/NanoPlot).

The sequences SEQ ID NO:4 and SEQ ID NO:5 were obtained from the genome sequencing.

Said sequence was automatically annotated using SEED viewer and the RAST 2.0 server (Rapid Annotation using Subsystem Technology) [Azid et al., 2008 BMC Genomics 9, 75; Overbeek et al., 2014 Nucleic Acid Research 42, D206-2014]. According to the data of this analysis, the sequence has a size of 6,096,876 base pairs and a G+C content of 66.2, having 2 contigs (those of SEQ ID NO:4 and SEQ ID NO:5) and 5808 coding sequences, and the number of RNAs was 57.

Likewise, the genome sequence was also annotated and analysed with the AntiSMASH server (MRNAix et al., 2011 Nucleic Acid Research doi: 10.1093/nar/gkr466) in order to analyse the existence of gene clusters for the biosynthesis of secondary metabolites. The results of these analyses revealed the presence of genes involved in plant growth-promoting mechanisms such as the presence of auxins and siderophores such as variochelin A and B, as well as terpene coding genes, which supports the good agronomic performance of the strain in the tests performed, which are shown in Example 2.

The genome sequence was also analysed to determine the taxonomic affiliation of the AFSI 2.2 strain and its precise identification, for which the average nucleotide identity (ANIb) was analysed using the JSpecies server [Ritcher and Mora, 2009, PNAS 106, 19126-19131; Ritcher et al., 2016; Bioinformatics 32(6), 929-931]. Likewise, digital DNA-DNA hybridisation (dDDH) values were calculated using the GGDC server (DSMZ genome-genome distance calculator (GGDC 2.1) (http://ggdc.dsmz.de/ggdc.php/). These values were calculated using formula 2 on the GGDC website. ANIb and dDDH values of the AFSI 2.2 strain were calculated with respect to the type strains of phylogenetically closest *Variovorax* species. The results of ANIb (Table 1) show that the AFSI 2.2 strain is phylogenetically closer to *V. paradoxus,* with an ANIb of 88.46%. The ANIb values of AFSI 2.2 with respect to other related species of the genus Variovorax range from 78.91 to 87.97%. In terms of dDDH values, the AFSI 2.2 strain presents 43.30% with respect to the phylogenetically closest species, *V. paradoxus,* and lower values with respect to the other *Variovorax* species being studied. These values are well below the threshold values recommended for the differentiation of bacterial species, which are ANIb values below 95-96% and a dDDH value below 70% [Goris *et al.,* 2007; Ritcher and Mora, 2009].

Therefore, the AFSI 2.2 strain belongs to a new species in the genus *Variovorax,* that has yet to be described for science, and this ensures its uniqueness and novelty.

**Table 1. ANIb values of the AFSI 2.2 strain with respect to phylogenetically related Variovorax species**

| | *Variovorax sp.* AFSI2.2 | *Variovorax gossypii* | *Variovorax boronicumulans* NBRC 103145 | *Variovorax guangxiensis* DSM 27352 | *Variovorax paradoxus* NBRC 15149 | *Variovorax soli* NBRC 106424 | *Variovorax beijingensis 502* [T] |
|---|---|---|---|---|---|---|---|
| *Variovorax sp.* AFSI2.2 | * | 83.14 *(55.23)* | 85.17 *(41.72)* | 84.88 *(45.48)* | 89.51 *(47.88)* | 79.22 *(35.03)* | 88.62 *(73.61)* |
| *Variovorax gossypii* | 83.06 *(53.92)* | * | 85.49 *(40.63)* | 90.37 *(47.38)* | 84.67 *(40.87)* | 78.45 *(31.10)* | 83.39 *(57.52)* |
| *Variovorax boronicumulans* NBRC 103145 | 84.00 *(66.65)* | 84.88 *(65.07)* | * | 85.89 *(73.86)* | 85.05 *(69.00)* | 78.87 *(57.17)* | 84.80 *(70.89)* |
| *Variovorax guangxiensis* DSM 27352 | 83.88 *(65.93)* | 89.85 *(69.73)* | 85.80 *(67.87)* | * | 84.91 *(69.46)* | 78.75 *(53.06)* | 84.68 *(69.33)* |
| *Variovorax paradoxus* NBRC 15149 | 88.46 *(74.39)* | 84.01 *(64.09)* | 85.06 *(67.34)* | 84.96 *(73.86)* | * | 78.75 *(57.21)* | 93.70 *(84.41)* |
| *Variovorax soli* NBRC 106424 | 78.91 *(63.11)* | 78.61 *(56.33)* | 79.73 *(65.04)* | 79.49 *(66.26)* | 79.55 *(66.74)* | * | 79.44 *(66.99)* |
| *Variovorax beijingensis* 502 [T] | 87.97 *(66.95)* | 83.05 *(53.71)* | 85.09 *(40.90)* | 84.92 *(43.88)* | 93.65 *(49.85)* | 79.09 *(34.08)* | * |

### 1.3. Deposit of biological matter in accordance with the Budapest Treaty

The AFSI 2.2 bacterial strain, the isolation and characterisation of which are disclosed in the present application, was deposited in the Coleccion Española de Cultivos Tipo (Spanish Type Culture Collection) (CECT), Parc Cientific Universitat de València, Catedrático Agustin Escardino, 9, 46980 Paterna, Valencia, Spain, under the conditions of the Budapest Treaty, acting as the depositor Azufrera y Fertilizantes Pallarès, SAU, whose address is at Avda. Europa 1-7, Pol. Ind. Constanti, 43120, Constanti, Tarragona, Spain. The deposit date and the number assigned are as follows:

| Bacterial strain | Abbreviated name | Accession number | Deposit date |
|---|---|---|---|
| *Variovorax* AFSI 2.2 | AFSI 2.2 | CECT 30738 | 25-10-2022 |

### Example 2. Tests with plants

To verify the effect of adding the isolated and selected AFSI 2.2 strain, tests involving the cultivation of several plants in a greenhouse and in the open air were performed, and the AFSI 2.2 strain was added to the irrigation water lacking or also including elemental sulphur. The specific tests performed are described below.

### 2.1. Greenhouse broccoli

A test was performed in a greenhouse, under non-controlled environmental conditions (under greenhouse conditions indicated as external environmental conditions) to evaluate the effectiveness of the AFSI 2.2 bacterium as a sulphur oxidiser and its effect on broccoli plants. The test was carried out from October 2020 to January 2021, with control plants without the delivery of bacteria or elemental sulphur.

24 seedlings (8 plants per treatment) of about 10 cm of the Parthenon variety, which had been acquired from the company Babyplant (Santomera, Murcia), were transplanted to pots with a height of 22 cm and a diameter of 22 cm. The pots were placed on two different tables, with 4 pots from each of the treatments being located on each table, and the plants were left to grow for a week, with drip irrigation directed to the root ball of between 1 and 3 litres of water/day applied every 24 hours. Next, the plants were kept in the same conditions, with the exception of starting their treatment following the scheme indicated in Table 2 below:

**Table 2.- Greenhouse broccoli test regime**

| Action | Days | Date | Treatment | | |
|---|---|---|---|---|---|
| | | | Control | AFSI 2.2 | AFSI 2.2 + S |
| Transplant | 0 | 7 October | 4x2 pots | 4x2 pots | 4x2 pots |
| Application A | 7 | 14 October | - | - | 50 ml |
| Application B | 14 | 21 October | - | 1 ml | 1 ml |
| Application C | 28 | 4 November | - | 1 ml | 1 ml |
| Assessment and cutting | 75 | 20 December | Yes | Yes | Yes |

- Application A: Microsul 40S (AFEPASA) mixed in water at 0.02% v/v. Total nutrient supplied: 0.4 g elemental sulphur + 0.02 g iron chelate. 50 ml were applied to the root ball by means of a syringe. No additional NPK (nitrogen, phosphorus, potassium) fertilisation was applied.
- Application B: 40 ml of AFSI 2.2 bacterial suspension in water, at a concentration of 10⁶ CFU/ml, were prepared. 1 ml of the suspension was applied to each pot, directly to the root ball with a syringe.
- Application C: 40 ml of AFSI 2.2 bacterial suspension in water, at a concentration of 10⁶ CFU/ml, were prepared. 1 ml of the suspension was applied to each pot, directly to the root ball with a syringe.
- Assessment and cutting: The height of each plant was measured (in centimetres), the part was cut level with the soil, and the whole part was weighed (in grams), and the head (which would be equivalent to the edible part) was the cut and weighed (in grams).

The results obtained with respect to the height and weight of the edible part are shown in Fig. 2, in which the mean value of the 8 pots of each treatment, as well as the standard deviation, can be seen.

As can be seen in said figure, by applying the AFSI 2.2 bacterium, the weight of the broccoli head increases; by adding an elemental sulphur supply, the weight of the head also increases when compared with the control. With respect to the height, it increases by applying the AFSI 2.2 bacterium, but the effect is greater if an elemental sulphur supplement is also applied.

### 2.2. Greenhouse cabbage

The greenhouse test of section 2.1 was repeated to evaluate the effectiveness of the AFSI 2.2 bacterium as a biostimulant for cabbage plant growth, specifically for the Green Lunar F1 variety, acquired from the company Babyplant (Santomera, Murcia). The test was carried out from February 2022 to May 2022, with control plants without the delivery of bacteria or elemental sulphur. For each treatment, 18 pots with a height of 22 cm and a diameter of 22 cm (6 pots x 3 tables) were used and seedlings of about 10 cm were transplanted to said pots. The test started from 1 February 2022 and treatment and irrigation regimes similar to those of section 2.1 were followed, except for the addition of phosphate and nitrogen in the first application, the application in which sulphur was supplied, as can be seen in Table 3 below:

**Table 3.- Greenhouse cabbage test regime**

| Action | Days | Date | Treatment | |
|---|---|---|---|---|
| | | | Control | AFSI 2.2 |
| Transplant | 0 | 1 February | 6x3 pots | 6x3 pots |
| Application A | 7 | 7 February | Yes | Yes |
| Application B | 14 | 15 February | - | 1 ml |
| Application C | 30 | 1 March | - | 1 ml |
| Application D | 45 | 14 March | | 1 ml |
| Application E | 60 | 28 March | | 1 ml |
| Application F | 75 | 11 April | | 1 ml |
| Assessment and cutting | 105 | 9 May | Yes | Yes |

- Application A: Soil preparation for all the plants including plants from control group:
   o Microsul 40S (AFEPASA) mixed in water at 0.02% v/v. Total nutrient supplied: 0.4 g elemental sulphur + 0.02 g iron chelate. 50 ml were applied to the root ball by means of a syringe.
   o Tricalcium Phosphate: mixed in water at 10% w/v. Total nutrients supplied: 0.05 g phosphorus and 0.22 g calcium (CaO). Applied to the root ball by means of a syringe.
   o Stilo Verde (SIPCAM Iberia, Valencia, Spain) mixed in water at 0.5% v/v. Total nutrients supplied: 0.2 g nitrogen. Applied to the root ball by means of a syringe.
- Applications B, C, D, E, and F: 60 ml of AFSI 2.2 bacterial suspension in water, at a concentration of 10⁸ CFU/ml, were prepared. 1 ml of the suspension was applied to each pot of the AFSI 2.2 treatment, directly to the root ball with a syringe.
- Assessment and cutting: The height of each plant was measured (in centimetres).

The results obtained with respect to the height and weight of the edible part are shown in Fig. 3, in which the mean value of the 18 pots of each treatment, as well as the standard deviation, can be seen.

As can be seen in said figure, by applying the AFSI 2.2 bacterium, an increase in cabbage plant growth, measured in height, can be seen.

### 2.3. Greenhouse radish and wheat

The greenhouse test of section 2.2 was repeated to evaluate the effectiveness of the AFSI 2.2 bacterium as a biostimulant of the growth of wheat plant (Hybiza variety) and radish (red round variety), both acquired as seeds from Fitó (Barcelona, Spain: www.semillasfito.es) and sown on 3 February 2022. The test was carried out from June 2022 to September 2022. For each treatment, 12 pots (6 pots x 2 tables) were used. Treatment started on 17 June 2022 and treatment regimes similar to that followed in section 2.2 were followed, as can be seen in Table 4 below:

**Table 4.- Greenhouse radish and wheat test regime**

| Action | Days | Date | Treatment | |
|---|---|---|---|---|
| | | | Control | AFSI 2.2 |
| Transplant | 0 | 17 June | 6x2 pots | 6x2 pots |
| Application A | 7 | 24 June | Yes | Yes |
| Application B | 14 | 1 July | - | 1 ml |
| Application C | 21 | 8 July | 1 ml | 1 ml |
| Application D | 28 | 15 July | - | 1 ml |
| Application E | 35 | 22 July | 1 ml | 1 ml |
| Assessment and harvesting | 42 | 29 July | Yes | Yes |

- Transplant: to pots with a height of 22 cm and a diameter of 22 cm.
- Application A: Soil preparation for all the plants including plants from control group:
   o Microsul 40S (AFEPASA) mixed in water at 0.02% v/v. Total nutrient supplied: 0.4 g elemental sulphur + 0.02 g iron chelate. 50 ml were applied to the root ball by means of a syringe.
   o Tricalcium Phosphate: mixed in water at 10% w/v. Total nutrients supplied: 0.05 g phosphorus and 0.22 g calcium (CaO). Applied to the root ball by means of a syringe.
   o Potassium nitrate mixed in water at 0.1% w/v. Total nutrients supplied: 0.007 g nitrogen and 0.012 g potassium (K₂O). Applied to the root ball by means of a syringe.
- Applications B and D: 60 ml of AFSI 2.2 bacterial suspension in water, at a concentration of 10⁸ CFU/ml, were prepared. 1 ml of the suspension was applied to each pot of the AFSI 2.2 treatment, aimed at the root ball with a syringe.
- Applications C and E: For all plants, including plants from control group: Potassium nitrate mixed in water at 0.1% w/v. Total nutrients supplied: 0.007 g nitrogen and 0.012 g potassium (K₂O). Applied to the root ball by means of a syringe.
- Assessment and cutting: The height of each whole plant was measured (in centimetres), the part was cut level with the soil, and the whole part was weighed (in grams) and then, in the case of radish, the part that would be equivalent to the edible part) was cut and weighed (in grams).

The results obtained with respect to the height and weight of the cut plant (in wheat) and the height of the cut plant and the weight of the edible part (radish) are shown in Figs. 4 and 5, respectively, in which the mean value of the 12 pots of each treatment, as well as the standard deviation, can be seen in each bar.

As can be seen in said figure, by applying the AFSI 2.2 bacterium, both the height and the weight increase, with the effect being particularly pronounced in the weight of cut wheat plants.

### 2.4. Open-air broccoli, lettuce, and zucchini

A test was performed in plants cultivated in the field, in the open air, to confirm the effectiveness of the AFSI 2.2 bacterium as a biostimulant, delivering it in liquid form together with the irrigation water and assessing its effect on zucchini, broccoli, and lettuce plants.

In all cases, and regardless of whether control plants or plants treated with AFSI 2.2 bacteria were going to be planted in the soil, an ecological, sulphate-free fertiliser was applied to the cultivation soil one week before the transplant to reproduce, as closely as possible, the common cultivation conditions for these plants. Said pretransplant application consisted of a dose of 5 kg/ha of UREA PRILL (ICL Iberia, Totana, Spain), which represents the application of a total of 2.3 kg nitrogen per hectare (ha).

In all cases, one week after the pretransplant application, seedlings of about 10 cm of each of the indicated species were transplanted to the soil in their corresponding cultivation fields to start the test. Sulphur applications were performed by means of drip irrigation, while the application of the bacteria was by means of a syringe, directly to the root ball. The plants were left to grow a total of 105 days, then they were harvested and the harvested products assessed. The particularities of the test for each plant are indicated below.

### 2.4.1. Zucchini.

- Variety: Icarus (supplied by Babyplant, Santomera, Murcia, Spain)
- Test location: Torre Pacheco (Murcia, Spain)
- Number of plants: 40 plants per treatment: control (without the application of AFSI 2.2 bacteria) or AFSI 2.2 (with the application of AFSI 2.2 bacteria)
- Planting density: 11111 plants/ha
- Test period: January to May 2022
- Treatment regimen: see Table 5

**Table 5.- Open-air zucchini test regime**

| Action | Days | Date | Treatment | |
|---|---|---|---|---|
| | | | Control | AFSI 2.2 |
| Pre-transplant fertilisation | -7 | | Yes | Yes |
| Transplant | 0 | 1 February | 40 seedlings | 40 seedlings |
| Application A | 14 | 15 February | - | Yes |
| Application B | 45 | 14 March | - | Yes |
| Application C | 75 | 11 April | - | Yes |
| Assessment and harvesting | 105 | 9 May | Yes | Yes |

- Applications A, B, and C: 60 ml of AFSI 2.2 bacterial suspension in water, at a concentration of 10⁸ CFU/ml, were prepared. 1 ml of the suspension was applied to each plant with a syringe, directly to the root ball.
- Assessment and harvesting: The part in each plant corresponding to its commercial product was harvested and total zucchini production was calculated by extrapolating it to kilograms/hectare (kg/ha).

The results obtained are shown in Fig. 6 which depicts production and standard deviation. As can be seen in said figure, applying the AFSI 2.2 bacterium causes an increase in the amount of zucchini produced.

### 2.4.2. Broccoli.

- Variety: Parthenon (supplied by Babyplant, Santomera, Murcia, Spain)
- Test location: Murcia, Spain
- Number of plants: 51 plants per treatment: control (without the application of AFSI 2.2 bacteria), AFSI 2.2 (with the application of AFSI 2.2 bacteria), and AFSI 2.2 +S (with the application of AFSI 2.2 bacteria and elemental sulphur)
- Test period: January to May 2022
- Treatment regimen: see Table 6

**Table 6.- Open-air broccoli test regime**

| Action | Days | Date | Treatment | | |
|---|---|---|---|---|---|
| | | | Control | AFSI 2.2 | AFSI 2.2 + S |
| Pre-transplant fertilisation | -7 | | Yes | Yes | Yes |
| Transplant | 0 | 1 February | 51 seedlings | 51 seedlings | 51 seedlings |
| Application A | 7 | 7 February | - | Yes | |
| Application B | 14 | 15 February | - | Yes | |
| Application C | 30 | 1 March | - | Yes | |
| Application D | 45 | 14 March | | | |
| Application E | 60 | 28 March | | | |
| Application F | 75 | 11 April | | | |
| Assessment and harvesting | 105 | 9 May | Yes | Yes | Yes |

- Applications A, C, and E: Organosul 40S (AFEPASA) mixed in water at 0.02% v/v. Total nutrient supplied: 0.2 g elemental sulphur + 0.04 g humic extract. Applied by means of drip irrigation.
- Applications B, D, and F: An AFSI 2.2 bacterial suspension in water, at a concentration of 10⁸ CFU/ml, was prepared. 1 ml of the suspension was applied to each plant with a syringe, directly to the root ball.
- Assessment and harvesting: The part in each plant corresponding to its commercial product was harvested (the head which is the edible part) and the harvested part of each plant was weighed.

The results obtained are shown in Fig. 7 which depicts the mean value of the weights obtained and standard deviation. As can be seen in said figure, applying the AFSI 2.2 bacterium causes an increase in the weight of the edible part of broccoli plants, with the increase being greater if an elemental sulphur source is also applied together with the bacterium.

### 2.4.3. Lettuce.

- Variety: Iceberg (supplied by Babyplant, Santomera, Murcia, Spain)
- Test location: Xilxes, Castellón, Spain
- Number of plants: 12 plants per treatment: control (without the application of AFSI 2.2 bacteria), AFSI 2.2 (with the application of AFSI 2.2 bacteria), and AFSI 2.2 +S (with the application of AFSI 2.2 bacteria and elemental sulphur)
- Test period: January to May 2022
- Treatment regimen: see Table 7

**Table 7.- Open-air lettuce test regime**

| Action | Days | Date | Treatment | | |
|---|---|---|---|---|---|
| | | | Control | AFSI 2.2 | AFSI 2.2 + S |
| Pre-transplant fertilisation | -7 | | Yes | Yes | Yes |
| Transplant | 0 | 1 February | 12 seedlings | 12 seedlings | 12 seedlings |
| Application A | 7 | 7 February | - | - | Yes |
| Application B | 14 | 15 February | - | Yes | Yes |
| Application C | 30 | 1 March | - | - | Yes |
| Application D | 45 | 14 March | | Yes | Yes |
| Application E | 60 | 28 March | | - | Yes |
| Application F | 75 | 11 April | | Yes | Yes |
| Assessment and harvesting | 105 | 9 May | Yes | Yes | Yes |

- Applications A, C, and E: Organosul 40S (AFEPASA) mixed in water at 0.02% v/v. Total nutrient supplied: 0.2 g elemental sulphur + 0.04 g humic extract. Applied by means of drip irrigation.
- Applications B, D, and F: An AFSI 2.2 bacterial suspension in water, at a concentration of 10⁸ CFU/ml, was prepared. 1 ml of the suspension was applied to each plant with a syringe, directly to the root ball.
- Assessment and harvesting: The vigour of each plant was measured in grams per heart and the mean value for each treatment group was calculated.

The results obtained are shown in Fig. 8 which depicts the mean of the values obtained in each group, as well as standard deviation. As can be seen in said figure, applying the AFSI 2.2 bacterium causes an increase of the vigour of lettuce plants, with the increase being greater if an elemental sulphur source is also applied together with the bacterium.

It can be concluded that treatment with AFSI 2.2 generally improves the growth of the plants to which it is applied and/or their yield with respect to the harvested part thereof, an effect which is generally favoured when elemental sulphur is also delivered, although the need or the benefit of said additional treatment can be determined by the conditions of the soil and the needs of each plant.

## Claims

1. A bacterium of the genus *Variovorax,* **characterised in that** it is capable of transforming elemental sulphur into sulphate, which is the bacterium deposited in the Colección Española de Cultivos Tipo (Spanish Type Culture Collection) (CECT) under number CECT 30738.

2. The bacterium according to claim 1, comprising in its genome:
a. a DNA fragment represented by SEQ ID NO:2; and/or
b. a DNA fragment represented by SEQ ID NO:3; and/or
c. a DNA fragment represented by SEQ ID NO:4, and/or
d. a DNA fragment represented by SEQ ID NO:5.

3. The bacterium according to any one of claims 1 or 2, having the capacity to promote plant growth.

4. A composition comprising the bacterium of any one of claims 1 to 3.

5. The composition of claim 4, comprising at least one biostimulant additional to the bacterium.

6. The composition of claim 5, comprising at least one additional biostimulant selected from the group of humic acids, fulvic acids, amino acids, peptides from protein hydrolysis, algae extracts, laminarin polysaccharides, alginate, carrageenan, polymeric chitosan, a chitosan oligomer, elemental aluminium, an aluminium salt, elemental cobalt, a cobalt salt, elemental sodium, a sodium salt, elemental selenium, a selenium salt, elemental silicon, a silicon salt, a mycorrhizal fungus, a biostimulant bacterium, or mixtures thereof.

7. The composition of any one of claims 4 to 6, which
is an aqueous composition,
or
is in non-particulate solid form or in the form of granules, powder, or pellets.

8. The composition of any one of claims 4 to 7, further comprising elemental sulphur.

9. Use of the bacterium of any one of claims 1 to 3 or of a composition of any one of claims 4 to 8, for promoting the growth of a plant and/or acting as a biostimulant for the plant.

10. Use according to claim 9, wherein the plant is a horticultural plant or a cereal.

11. Use according to claim 10, wherein the plant
belongs to the *Brassicaceae* family,
or
is selected from the group of broccoli, cabbage, radish, lettuce, zucchini, or wheat.

12. Use according to any one of claims 9 to 11, wherein the composition is the irrigation water for the plant or the nutrient solution for a hydroponically cultivated plant or is mixed therewith for delivery to the plant.

13. Use according to any one of claims 9 to 12, wherein elemental sulphur is present in the soil where the plant grows or in the nutrient solution delivered to a hydroponically cultivated plant.

14. A method for the modification and/or restoration of a soil, comprising at least one step in which a bacterium of any one of claims 1 to 3 or a composition of any one of claims 4 to 8 is provided thereto.

15. The method according to claim 14, wherein the soil is alkaline, limestone, and/or saline soil.

16. The method according to any one of claims 14 or 15, wherein the modification of soil is selected from the group of enrichment with sulphur salts, solubilisation of phosphorus salts, reduction of pH, increase of conductivity, increase of porosity, or mixtures of the above.

17. The method according to any one of claims 14 to 16, comprising one or more steps in which elemental sulphur is delivered to the soil.

18. The method according to claim 17, wherein a composition in non-particulate solid form or in the form of granules, powder or pellets is provided to the soil.

19. The use according to any one of claims 9 to 13 or the method according to claim 17, wherein the elemental sulphur is delivered in one or more applications separated in time from the applications of the bacterium of any one of claims 1 to 3 or a composition of any one of claims 4 to 8.

## Patentansprüche

1. Bakterium der Gattung *Variovorax,* **dadurch gekennzeichnet, dass** es in der Lage ist, elementaren Schwefel in Sulfat umzuwandeln, wobei es sich um das Bakterium handelt, das in der Colección Española de Cultivos Tipo (Spanische Sammlung von Typenkulturen) (CECT) unter der Nummer CECT 30738 hinterlegt ist.

2. Bakterium nach Anspruch 1, das in seinem Genom Folgendes umfasst:
a. ein DNA-Fragment, dargestellt durch SEQ ID NO:2; und/oder
b. ein DNA-Fragment, dargestellt durch SEQ ID NO:3; und/oder
c. ein DNA-Fragment, dargestellt durch SEQ ID NO:4, und/oder
d. ein DNA-Fragment, dargestellt durch SEQ ID NO:5.

3. Bakterium nach einem der Ansprüche 1 oder 2, das die Fähigkeit besitzt, das Pflanzenwachstum zu fördern.

4. Zusammensetzung, umfassend das Bakterium nach einem der Ansprüche 1 bis 3.

5. Zusammensetzung nach Anspruch 4, die zusätzlich zu dem Bakterium mindestens ein Biostimulans umfasst.

6. Zusammensetzung nach Anspruch 5, umfassend mindestens ein zusätzliches Biostimulans, ausgewählt aus der Gruppe bestehend aus Huminsäuren, Fulvinsäuren, Aminosäuren, Peptiden aus der Proteinhydrolyse, Algenextrakten, Laminarin-Polysacchariden, Alginat, Carrageenan, polymerem Chitosan, einem Chitosan-Oligomer, elementarem Aluminium, einem Aluminiumsalz, elementarem Kobalt, einem Kobaltsalz, elementarem Natrium, einem Natriumsalz, elementarem Selen, einem Selensalz, elementarem Silizium, einem Siliziumsalz, einem Mykorrhizapilz, einem biostimulierenden Bakterium oder Mischungen davon.

7. Zusammensetzung nach einem der Ansprüche 4 bis 6, die
eine wässrige Zusammensetzung ist,
oder
in nicht partikelförmiger fester Form oder in Form von Granulat, Pulver oder Pellets vorliegt.

8. Zusammensetzung nach einem der Ansprüche 4 bis 7, die ferner elementaren Schwefel umfasst.

9. Verwendung des Bakteriums nach einem der Ansprüche 1 bis 3 oder einer Zusammensetzung nach einem der Ansprüche 4 bis 8 zur Förderung des Wachstums einer Pflanze und/oder zur Wirkung als Biostimulans für die Pflanze.

10. Verwendung nach Anspruch 9, wobei die Pflanze eine Gartenbaupflanze oder ein Getreide ist.

11. Verwendung nach Anspruch 10, wobei die Pflanze
zur Familie der *Brassicaceae* gehört,
oder
ausgewählt ist aus der Gruppe von Brokkoli, Kohl, Rettich, Salat, Zucchini oder Weizen.

12. Verwendung nach einem der Ansprüche 9 bis 11, wobei die Zusammensetzung das Bewässerungswasser für die Pflanze oder die Nährlösung für eine hydroponisch kultivierte Pflanze ist oder mit diesen zur Abgabe an die Pflanze vermischt wird.

13. Verwendung nach einem der Ansprüche 9 bis 12, wobei elementarer Schwefel in dem Boden, in dem die Pflanze wächst, oder in der Nährlösung, die einer hydroponisch kultivierten Pflanze zugeführt wird, vorhanden ist.

14. Verfahren zur Modifizierung und/oder Wiederherstellung eines Bodens, umfassend mindestens einen Schritt, in dem ein Bakterium nach einem der Ansprüche 1 bis 3 oder eine Zusammensetzung nach einem der Ansprüche 4 bis 8 diesem zugeführt wird.

15. Verfahren nach Anspruch 14, wobei der Boden alkalischer, kalkhaltiger und/oder salzhaltiger Boden ist.

16. Verfahren nach einem der Ansprüche 14 oder 15, wobei die Modifizierung des Bodens aus der Gruppe Anreicherung mit Schwefelsalzen, Solubilisierung von Phosphorsalzen, Senkung des pH-Werts, Erhöhung der Leitfähigkeit, Erhöhung der Porosität oder Mischungen der oben genannten ausgewählt ist.

17. Verfahren nach einem der Ansprüche 14 bis 16, umfassend einen oder mehrere Schritte, in denen elementarer Schwefel an den Boden abgegeben wird.

18. Verfahren nach Anspruch 17, wobei dem Boden eine Zusammensetzung in nicht partikelförmiger fester Form oder in Form von Granulat, Pulver oder Pellets zugeführt wird.

19. Verwendung nach einem der Ansprüche 9 bis 13 oder Verfahren nach Anspruch 17, wobei der elementare Schwefel in einer oder mehreren Anwendungen zugeführt wird, die zeitlich von den Anwendungen des Bakteriums nach einem der Ansprüche 1 bis 3 oder einer Zusammensetzung nach einem der Ansprüche 4 bis 8 getrennt sind.

## Revendications

1. Bactérie du genre *Variovorax,* **caractérisée en ce qu'**elle est capable de transformer le soufre élémentaire en sulfate, qui est la bactérie déposée auprès de la Colección Española de Cultivos Tipo (collection espagnole de cultures types) (CECT) sous le numéro CECT 30738.

2. Bactérie selon la revendication 1, comprenant dans son génome :
a. un fragment d'ADN représenté par la SEQ ID NO : 2 ; et/ou
b. un fragment d'ADN représenté par la SEQ ID NO : 3 ; et/ou
c. un fragment d'ADN représenté par la SEQ ID NO : 4, et/ou
d. un fragment d'ADN représenté par la SEQ ID NO : 5.

3. Bactérie selon l'une quelconque des revendications 1 ou 2, ayant la capacité de favoriser la croissance d'une plante.

4. Composition comprenant la bactérie selon l'une quelconque des revendications 1 à 3.

5. Composition selon la revendication 4, comprenant au moins un biostimulant en plus de la bactérie.

6. Composition selon la revendication 5, comprenant au moins un biostimulant supplémentaire choisi dans le groupe des acides humiques, des acides fulviques, des acides aminés, des peptides issus de l'hydrolyse de protéines, des extraits d'algues, des polysaccharides de laminarine, de l'alginate, du carraghénane, d'un chitosane polymère, d'un oligomère de chitosane, de l'aluminium élémentaire, d'un sel d'aluminium, du cobalt élémentaire, d'un sel de cobalt, du sodium élémentaire, d'un sel de sodium, du sélénium élémentaire, d'un sel de sélénium, du silicium élémentaire, d'un sel de silicium, d'un champignon mycorhizien, d'une bactérie biostimulante, ou de mélanges de ceux-ci.

7. Composition selon l'une quelconque des revendications 4 à 6, qui
est une composition aqueuse,
ou
est sous forme solide non particulaire ou sous la forme de granulés, de poudre ou de pastilles.

8. Composition selon l'une quelconque des revendications 4 à 7, comprenant en outre du soufre élémentaire.

9. Utilisation de la bactérie selon l'une quelconque des revendications 1 à 3 ou d'une composition selon l'une quelconque des revendications 4 à 8, pour favoriser la croissance d'une plante et/ou agir comme biostimulant pour la plante.

10. Utilisation selon la revendication 9, dans laquelle la plante est une plante horticole ou une céréale.

11. Utilisation selon la revendication 10, dans laquelle la plante
appartient à la famille des *Brassicaceae,*
ou
est choisie dans le groupe du brocoli, du chou, du radis, de la laitue, de la courgette ou du blé.

12. Utilisation selon l'une quelconque des revendications 9 à 11, dans laquelle la composition est l'eau d'irrigation pour la plante ou la solution nutritive pour une plante cultivée en hydroponie ou est mélangée à celle-ci pour une administration à la plante.

13. Utilisation selon l'une quelconque des revendications 9 à 12, dans laquelle du soufre élémentaire est présent dans le sol où la plante pousse ou dans la solution nutritive administrée à une plante cultivée en hydroponie.

14. Procédé de modification et/ou de restauration d'un sol, comprenant au moins une étape au cours de laquelle une bactérie selon l'une quelconque des revendications 1 à 3 ou une composition selon l'une quelconque des revendications 4 à 8 est fournie.

15. Procédé selon la revendication 14, dans lequel le sol est un sol alcalin, calcaire et/ou salin.

16. Procédé selon l'une quelconque des revendications 14 ou 15, dans lequel la modification du sol est choisie dans le groupe de l'enrichissement en sels de soufre, de la solubilisation de sels de phosphore, de la réduction du pH, de l'augmentation de la conductivité, de l'augmentation de la porosité, ou des mélanges de ce qui précède.

17. Procédé selon l'une quelconque des revendications 14 à 16, comprenant une ou plusieurs étapes au cours desquelles du soufre élémentaire est apporté au sol.

18. Procédé selon la revendication 17, dans lequel une composition sous forme solide non particulaire ou sous la forme de granulés, de poudre ou de pastilles est fournie au sol.

19. Utilisation selon l'une quelconque des revendications 9 à 13 ou procédé selon la revendication 17, dans lequel le soufre élémentaire est apporté en une ou plusieurs applications séparées dans le temps des applications de la bactérie selon l'une quelconque des revendications 1 à 3 ou d'une composition selon l'une quelconque des revendications 4 à 8.
